# EUROPEAN PATENT APPLICATION

(11) **EP 4 316 543 A1**
(43) Date of publication of application: **07.02.2024**
(21) Application number: 22780967.0
(22) Date of filing: 29.03.2022
(51) Int. Cl.: A61M 1/00

(54) **SUCTION OBJECT GUIDING DEVICE AND SUCTION OBJECT SUCTION SYSTEM**

(30) Priority: 31.03.2021 JP 2021062367
(71) Applicant: Ishikita, Naoyuki, Kashiwazaki-shi, Niigata 945-8585 (JP)
(72) Inventor: Ishikita, Naoyuki, Kashiwazaki-shi, Niigata 945-8585 (JP)
(74) Representative: Schmid, Wolfgang
(86) International application number: PCT/JP2022/015621
(87) International publication number: WO 2022/210756

(57) **Abstract**

To more easily perform sputum suctioning at a low degree of invasion. An aspirate-guiding apparatus 100 for guiding an aspirate from a living body to an upper respiratory tract includes an aspirate outlet 101 through which the aspirate is dischargeable, a patient connection port 103 that is connectable to a patient, an intake port 104 that is connectable to an intake apparatus, and a switching valve that switches a connection source of the patient connection port to the aspirate outlet or the intake port. The aspirate-guiding apparatus 100 further includes a casing 102 that includes the aspirate outlet 101, the patient connection port 103, and the intake port 104. The switching valve is slidably provided in the casing and is capable of switching the connection source of the patient connection port to the aspirate outlet or the intake port.

## Description

### Technical Field

The present disclosure relates to an aspirate-guiding apparatus and an aspirate-suctioning system.

### Background Art

In some cases, aspirates in a human body such as sputum, saliva, and nasal discharge that are accumulated in the pharynx are difficult to come out from the body and to be swallowed through a cough and swallowing because of aging or illness. Such an aspirate that enters the trachea causes, for example, difficulty breathing, suffocation, pneumonia, or atelectasis. For this reason, it is necessary to suction and remove the aspirate by using, for example, a suction catheter that is connected to a suction tube of a suction apparatus.

When the aspirate such as sputum is suctioned by the suction catheter, the effects of suctioning are not sufficiently exerted unless an end portion of the suction catheter reaches the aspirate. For this reason, there is a concern that the suction catheter is inserted into the lower respiratory tract, and the respiratory tract is damaged. An example of a technique related to sputum suctioning is a collecting device that is disclosed in PTL 1 and that suctions and collects sputum by using a suction catheter after the sputum in the lung is guided to the upper respiratory tract by using mechanical insufflation exsufflation (referred to below as MI-E) in which negative pressure is applied after the pressure in the lung of a patient is adjusted to positive pressure by using a machine.

### Citation List

### Patent Literature

PTL 1: Japanese Unexamined Patent Application Publication (Translation of PCT Application) No. 2013-524202 (paragraph [0017] in a specification)

### Summary of Invention

### Technical Problem

In the sputum suctioning by using the mechanical insufflation exsufflation (MI-E), the sputum is guided to the upper respiratory tract, and accordingly, the sputum suctioning can be unlikely to cause the suction catheter to damage the respiratory tract by at a low degree of invasion. However, the sputum suctioning by using the mechanical insufflation exsufflation (MI-E) needs a technique with skilled machine operations and settings. For this reason, it is preferable that the aspirate such as sputum be more easily guided to the upper respiratory tract.

The present disclosure has been accomplished in view of the above problems, and it is an object of the present disclosure to more easily guide an aspirate to the upper respiratory tract.

### Solution to Problem

According to an aspect the present disclosure, an aspirate-guiding apparatus for guiding an aspirate from a living body to an upper respiratory tract includes an aspirate outlet through which the aspirate is dischargeable, a patient connection port that is connectable to a patient, an intake port that is connectable to an intake apparatus, and a switching valve that switches a connection source of the patient connection port to the aspirate outlet or the intake port.

According to the aspect of the present disclosure, the switching valve can easily switch the connection source of the patient connection port of the aspirate-guiding apparatus that is connected to a suction apparatus and the intake apparatus. For this reason, the patient connection port is connected to a wearable ventilation member for positive pressure ventilation, the switching valve performs an operation to switch the connection source of the patient connection port, and vacuuming for guiding the aspirate to the upper respiratory tract under negative pressure can be easily performed.

According to another aspect of the present disclosure, an aspirate-suctioning system for suctioning an aspirate from a living body includes a suction apparatus that includes a suction tube, the aspirate-guiding apparatus that is connected to an end of the suction tube and that is described above, and the intake apparatus that is connected to the intake port of the aspirate-guiding apparatus.

According to the other aspect of the present disclosure, the patient connection port of an aspirate-guiding tube that is connected to the suction apparatus and the intake apparatus is connected to the wearable ventilation member, the positive pressure ventilation is performed, the switching valve subsequently performs the operation to switch the connection source of the patient connection port such that these are in communication with each other. Consequently, vacuuming for guiding the aspirate to the upper respiratory tract under negative pressure can be easily performed.

### Advantageous Effects of Invention

According to the present disclosure, the aspirate can be more easily guided to the upper respiratory tract.

### Brief Description of Drawings

[Fig. 1] Fig. 1 is a plan view of a schematic structure of an aspirate-guiding apparatus according to an embodiment of the present disclosure.
[Fig. 2] Fig. 2(A) is a front view of a connection source switch that is included in the aspirate-guiding apparatus according to an embodiment of the present disclosure, Fig. 2(B) is a plan view of the connection source switch, Fig. 2(C) is a bottom view of the connection source switch, and Fig. 2(D) illustrates the structure of a switching valve inside the connection source switch.
[Fig. 3] Fig. 3(A) is a perspective view of an aspirate-guiding tube that is disassembled and that is included in the aspirate-guiding apparatus according to an embodiment of the present disclosure, and Fig. 3(B) is a sectional view taken along line A-A in Fig. 3(A).
[Fig. 4] Fig. 4(A) and Fig. 4(B) illustrate the motion of the switching valve of the aspirate-guiding apparatus according to an embodiment of the present disclosure to switch the connection source of a patient connection port.
[Fig. 5] Fig. 5 illustrates a schematic structure of an aspirate-suctioning system that uses the aspirate-guiding apparatus according to an embodiment of the present disclosure.
[Fig. 6] Fig. 6 illustrates another aspect of the schematic structure of the aspirate-suctioning system that uses the aspirate-guiding apparatus according to an embodiment of the present disclosure.
[Fig. 7] Fig. 7 illustrates another aspect of the schematic structure of the aspirate-suctioning system that uses the aspirate-guiding apparatus according to an embodiment of the present disclosure.
[Fig. 8] Fig. 8 is a perspective view of an aspirate-guiding apparatus according to another embodiment of the present disclosure.
[Fig. 9] Fig. 9 is a sectional view taken along line B-B in Fig. 7.
[Fig. 10] Fig. 10(A) is a perspective view of a switching valve that is included in the aspirate-guiding apparatus according to another embodiment of the present disclosure, and Fig. 10(B) is a sectional view taken along line C-C in Fig. 10(A).
[Fig. 11] Fig. 11(A) and Fig. 11(B) illustrate the motion of the aspirate-guiding apparatus according to another embodiment of the present disclosure.
[Fig. 12] Fig. 12 illustrates a schematic structure of an aspirate-suctioning system that uses the aspirate-guiding apparatus according to another embodiment of the present disclosure.
[Fig. 13] Fig. 13 illustrates another aspect of the schematic structure of the aspirate-suctioning system that uses the aspirate-guiding apparatus according to another embodiment of the present disclosure.
[Fig. 14] Fig. 14 illustrates another aspect of the schematic structure of the aspirate-suctioning system that uses the aspirate-guiding apparatus according to another embodiment of the present disclosure.

### Description of Embodiments

Preferred embodiments of the present disclosure will hereinafter be described in detail. The embodiments described below do not unreasonably limit the content of the present disclosure described in claims. All components described according to these embodiments are not necessarily essential as solutions of the present disclosure.

In the description below, words that represent "up", "down", "left" and "right" are used for convenience of description and do not limit methods of use or aspects of use. Words such as "first" and "n-th" (n is an integer) subsequent to the "first" described in the present specification and the claims are used as identification words for distinguishing different elements and do not represent a specific order or priority.

Words that are used for the description below are used merely to describe specific embodiments and do not limit the range of the present disclosure. If whether a component in an aspect described in the present specification and the claims have a singular form or a plural form is contextually unclear, the component may have a plural form. The words "and/or" represent one or more elements of relevant elements that are enumerated or all combinations thereof, and these are included. The words "includes", "including", "comprises", and/or "comprising" described in the present specification and the claims specify the presence of a feature, a motion, an element, and a step. However, one or more features, motions, elements, steps, and/or the presence or addition of the group thereof are excluded.

### (First Embodiment)

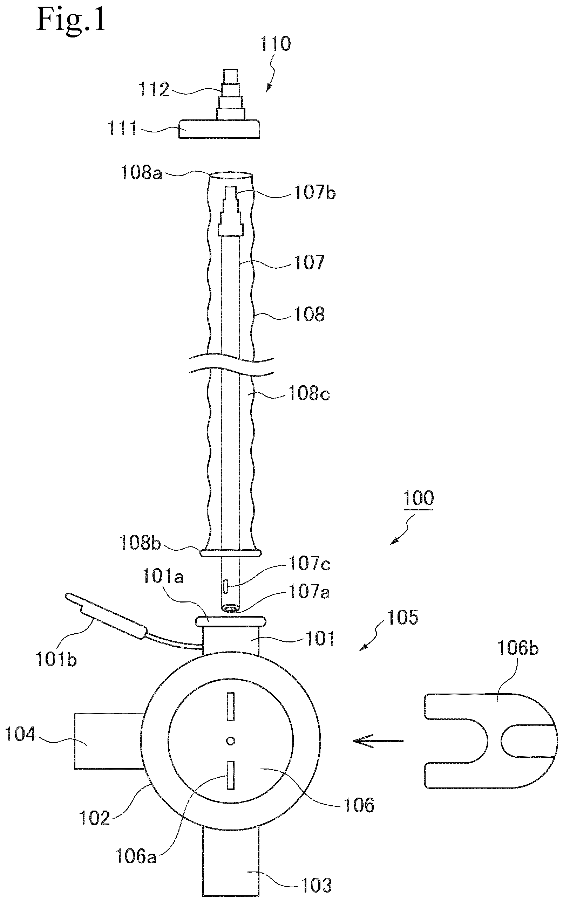

A schematic structure of an aspirate-guiding tube according to an embodiment of the present disclosure will be described with reference to the drawings. Fig. 1 is a plan view of a schematic structure of an aspirate-guiding apparatus according to an embodiment of the present disclosure.

An aspirate-guiding apparatus 100 according to the present embodiment is used to guide an aspirate (a biological fluid such as sputum) from the respiratory tract including the lower respiratory tract of a patient that is a living body to the upper respiratory tract and to suction and remove the aspirate. The aspirate-guiding apparatus 100 is connected to a suction apparatus that is used when the aspirate is suctioned and removed and an intake apparatus that is used for positive pressure ventilation before and after the aspirate is suctioned and removed. As illustrated in Fig. 1, the aspirate-guiding apparatus 100 includes an aspirate-guiding tube 110, a connection source switch 105, a suction catheter 107, and a cover member 108.

The aspirate-guiding tube 110 is used as a member that is mounted at an end of a suction tube connected to the suction apparatus (a stationary or portable sputum suction device) and that guides the aspirate to the upper respiratory tract by using the suction force (negative pressure) of the suction apparatus. As for the aspirate-guiding tube 110, a connection tube 112 that corresponds to a suction connection portion that is connected to the suction tube is provided on a surface of a guiding tube body 111. According to the present embodiment, the connection tube 112 can be engaged with a base end portion 107b of the suction catheter 107 inside and connected to the suction catheter 107. The structure of the aspirate-guiding tube 110 will be described in detail later.

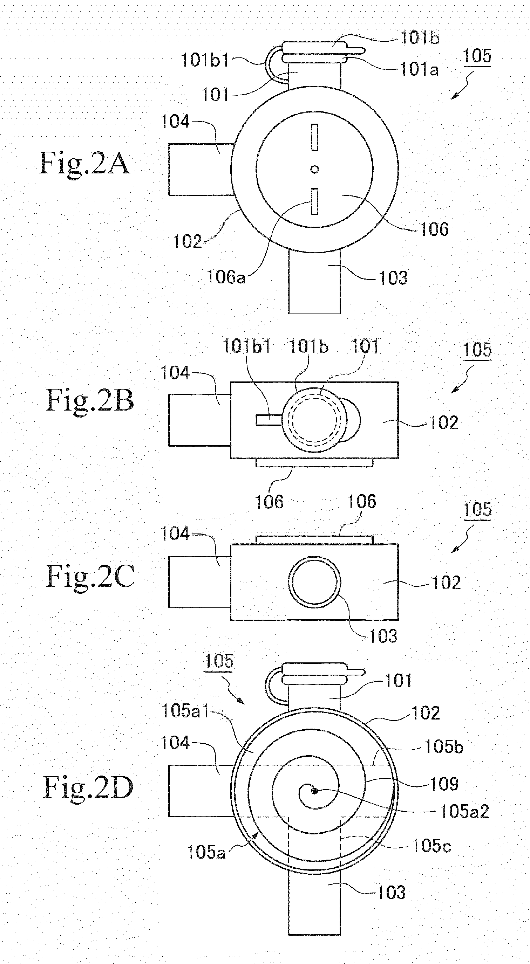

The connection source switch 105 has a function of switching the connection source of a patient connection port 103 to an aspirate outlet 101 or an intake port 104 by using a switching valve 105a (see Fig. 2(D)) that is slidably provided in a casing 102. As illustrated in Fig. 1, the connection source switch 105 includes the aspirate outlet 101, the casing 102, the patient connection port 103, the intake port 104, and an operation unit 106.

The casing 102 is a columnar member composed of resin. As illustrated in Fig. 1, the aspirate outlet 101 is provided at the upper end of the casing 102, and the patient connection port 103 that has an inner diameter of, for example, about 15 mm and that is tubular is provided at the lower end. The aspirate outlet 101 functions as an outlet through which the aspirate is discharged to the outside via the aspirate-guiding tube 110 or the suction catheter 107 that is connected to the aspirate-guiding tube 110. According to the present embodiment, an engagement projection portion 101a that is annular is provided at the upper end of the aspirate outlet 101 and is capable of opening and closing by using a lid portion 101b. The patient connection port 103 functions a port that is connected to a tubular end of a wearable ventilation member such as a tracheal tube or a tracheal cannula that is worn on the patient to transmit the negative pressure from the suction apparatus to the respiratory tract of the patient.

According to the present embodiment, as illustrated in Fig. 1, the casing 102 is provided such that the aspirate outlet 101 and the patient connection port 103 face each other. As for the casing 102, the intake port 104 that is connectable to the intake apparatus is provided between the aspirate outlet 101 and the patient connection port 103. As illustrated in Fig. 1, the intake port 104 is a tubular member that is provided so as to branch in a perpendicular direction from a side surface of the casing 102.

The intake port 104 functions as a port that is connected to the intake apparatus during the positive pressure ventilation against the patient by using the intake apparatus of various intake apparatuses such as a manual elastic bag and a mechanical intake apparatus. According to the present embodiment, the intake port 104 is a tubular member that is provided so as to branch from the side surface of the casing 102. However, the aspects of the intake port 104 are not limited to those of a tubular member. That is, an example of the "intake port" that is provided in the casing 102 may be a hole that is formed in a surface of the casing 102 into which an intake port of the intake apparatus is fitted.

The operation unit 106 has a function of performing an operation to switch the connection source of the patient connection port 103 by causing the switching valve 105a (see Fig. 2(D)) in the casing 102 to rotate along the casing 102. That is, the switching valve 105a is capable of switching the connection source of the patient connection port 103 to the aspirate outlet 101 or the intake port 104 by using the operation unit 106.

According to the present embodiment, the operation unit 106 performs an operation to rotate the switching valve 105a with a key wedge 106b that is rotatable inserted into an engagement hole 106a that corresponds to a keyhole. The key wedge 106b also has a function of separating tubes from each other by using wedge principle in a manner in which the key wedge 106b is inserted into a tube connection portion at which the patient connection port 103 of the connection source switch 105 is connected to the tubular end of the wearable ventilation member such as a tracheal tube or a tracheal cannula. The operation unit 106 may perform another operation other than the rotating operation with the key wedge 106b inserted into the engagement hole 106a, provided that the operation to rotate the switching valve 105a in the casing 102 can be performed. The operation of the operation unit 106 to switch the switching valve 105a will be described in detail later.

The suction catheter 107 is a tubular member that is composed of, for example, resin and that is used when the aspirate such as a biological fluid is suctioned and removed. The suction catheter 107 has an end portion 107a that opens, and an air vent 107c that has a slit shape is provided on a side surface near the end portion 107a. According to the present embodiment, the suction catheter 107 is sized such that the base end portion 107b can be fitted into the connection tube 112 of the aspirate-guiding tube 110. As for the suction catheter 107, the base end portion 107b is fitted into the connection tube 112 of the aspirate-guiding tube 110, and the end portion 107a is inserted from the aspirate outlet 101. In this way, the suction catheter 107 connects the connection tube 112 that corresponds to the suction connection portion and the aspirate outlet 101 to each other.

The thickness of the suction catheter 107 has variations and is not particularly limited. However, in order to make the negative pressure from the suction apparatus likely to be transmitted to the respiratory tract of the patient via the connection source switch 105, the diameter of the suction catheter 107 is preferably at least 5 mm or more. Similarly, the inner diameter of the connection tube 112 of the aspirate-guiding tube 110 that is connected to the suction catheter 107 is preferably 5 mm or more. In the case where the suction catheter 107 is mounted and used after the sputum is lifted up to an upper portion of the respiratory tract, the suction catheter 107 may be thinner than the inner diameter of the connection tube 112. The suction catheter 107 is at least thinner than the inner diameter of the wearable ventilation member such as a tracheal tube that is worn on the patient that corresponds to a connection destination.

The cover member 108 is a tubular member that is composed of a transparent resin sheet such as PMMA (acryl), PET (polyethylene terephthalate), or PC (polycarbonate) and that is flexible. According to the present embodiment, the cover member 108 is mounted such that an end portion 108a is inserted into the connection tube 112 of the aspirate-guiding tube 110 so as to cover the outer circumference of the base end portion 107b of the suction catheter 107. The cover member 108 is mounted such that a base end portion 108b that is elastic is engaged with the engagement projection portion 101a of the aspirate outlet 101 of the connection source switch 105. The cover member 108 is mounted between the aspirate-guiding tube 110 and the aspirate outlet 101, connects the aspirate-guiding tube 110 and the aspirate outlet 101 to each other, and forms a closed passage 108c that contains the suction catheter 107.

As for the aspirate-guiding apparatus 100 according to the present embodiment, the aspirate-guiding tube 110 that is mounted at the end of the suction tube that is connected to the suction apparatus is connected to the aspirate outlet 101 of the connection source switch 105 with the suction catheter 107 and the cover member 108 interposed therebetween. The connection source switch 105 includes the switching valve 105a that is capable of switching the connection source of the patient connection port 103 to the aspirate outlet 101 or the intake port 104.

For this reason, the connection source switch 105 switches the connection source of the patient connection port 103 to the aspirate outlet 101, the aspirate is consequently guided to the upper respiratory tract by using the suction force (negative pressure) of the suction apparatus, and the aspirate can be subsequently suctioned and removed by the suction catheter 107. The connection source switch 105 switches the connection source of the patient connection port 103 to the intake port 104, air or oxygen can be consequently introduced into the lung of the patient, and the positive pressure ventilation can be performed. According to the present embodiment, the suction catheter 107 and the cover member 108 can be installed and removed between the aspirate-guiding tube 110 and the connection source switch 105. For this reason, after the aspirate is suctioned and removed, and after the positive pressure ventilation, the suction catheter 107 and the cover member 108 can be removed, discarded, and replaced with new ones.

The structure of the connection source switch that includes the switching valve that is included in the aspirate-guiding apparatus according to an embodiment of the present disclosure will now be described in detail with reference to the drawings. Fig. 2(A) is a front view of the connection source switch that is included in the aspirate-guiding apparatus according to an embodiment of the present disclosure, Fig. 2(B) is a plan view of the connection source switch, Fig. 2(C) is a bottom view of the connection source switch, and Fig. 2(D) illustrates the structure of the switching valve inside the connection source switch. Fig. 2(D) illustrates a state in which the operation unit 106 is removed from the casing 102.

As for the connection source switch 105 according to the present embodiment, the aspirate outlet 101, the patient connection port 103, and the intake port 104 are mounted on the casing 102 that contains the switching valve 105a, and these components are integrally molded. That is, as for the connection source switch 105, these components are integrally molded by injection-molding thermoplastic resin such as polyethylene (PE), polypropylene (PP), polyvinyl chloride (PVC), ABS resin, or acrylic resin (PMMA).

As illustrated in Fig. 2(A) and Fig. 2(B), the aspirate outlet 101 is provided at the upper end of the casing 102, and the aspirate outlet 101 is capable of opening and closing by using the lid portion 101b that is connected with a connecting portion 101 b1 that is connected to the side surface interposed therebetween. As illustrated in Fig. 2(A) and Fig. 2(C), the patient connection port 103 that is tubular is provided at the lower end of the casing 102 and is connectable to the tubular end of the wearable ventilation member such as a tracheal tube or a tracheal cannula that is worn on the patient. The intake port 104 that is tubular is provided on the side surface of the casing 102 so as to branch in the perpendicular direction from the side surface of the casing 102.

As illustrated in Fig. 2(D), in the casing 102, the switching valve 105a that includes a valve member 105a1 that has a columnar shape and that has a suction ventilation path 105b and an intake ventilation path 105c is rotatably provided in the casing 102. According to the present embodiment, the switching valve 105a is slidable in the casing 102 so as to rotate about a central portion 105a2 of the valve member 105a1.

As for the switching valve 105a according to the present embodiment, as illustrated in Fig. 2(D), the suction ventilation path 105b extends through the valve member 105a1 in a radial direction, and the intake ventilation path 105c branches from the suction ventilation path 105b in the vertical direction. That is, the switching valve 105a has a T-shaped ventilation path that is formed by the suction ventilation path 105b and the Intake ventilation path 105c in the valve member 105a1. The switching valve 105a according to the present embodiment has the T-shaped ventilation path in the valve member 105a1 that has a columnar shape and that is composed of, for example, thermoplastic resin described above, but a T-shaped tubular path, for example, may be provided in a columnar case.

According to the present embodiment, as illustrated in Fig. 2(D), the switching valve 105a includes a flat spiral spring 109 that spirally connects the central portion 105a2 of the valve member 105a1 and the inside of the casing 102. The flat spiral spring 109 rotatably urges the switching valve 105a. The flat spiral spring 109 is thus provided, and consequently, the suction ventilation path 105b is directed in the horizontal direction and can be in communication with the intake port 104 in a neutral, normal state in which the restoring force of the flat spiral spring 109 does not act as illustrated in Fig. 2(D). In addition, as for the switching valve 105a, the valve member 105a1 is disposed in the casing 102 such that the intake ventilation path 105c is directed downward in the vertical direction so as to be capable of being in communication with the patient connection port 103. As for the switching valve 105a, the valve member 105a1 is thus disposed in the casing 102, and the connection source of the patient connection port 103 is the intake port 104. The motion of the switching valve 105a to switch the connection source of the patient connection port 103 will be described in detail later.

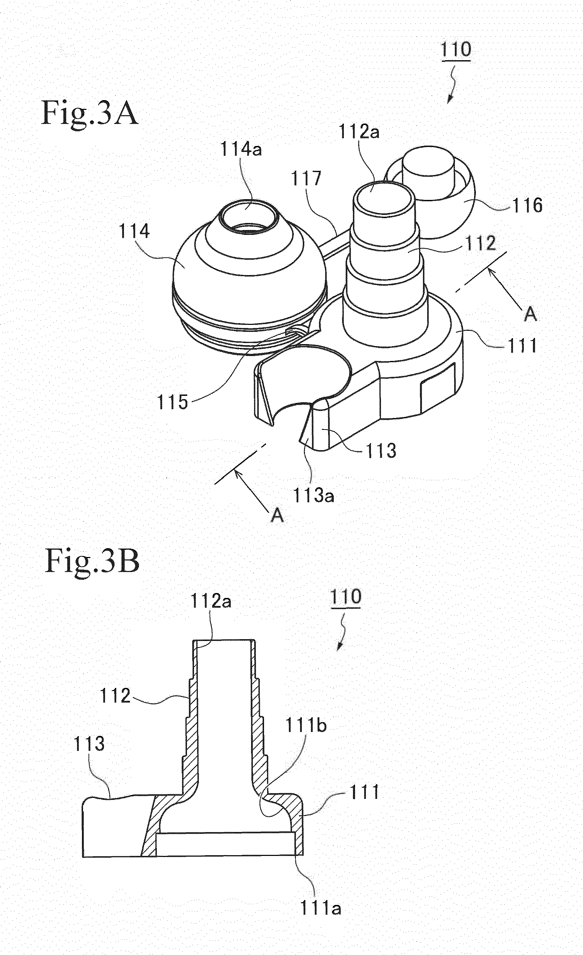

The structure of the aspirate-guiding tube that is included in the aspirate-guiding apparatus according to the present embodiment will now be described with reference to the drawings. Fig. 3(A) is a perspective view of the aspirate-guiding tube that is disassembled and that is included in the aspirate-guiding apparatus according to an embodiment of the present disclosure, and Fig. 3(B) is a sectional view taken along line A-A in Fig. 3(A).

As illustrated in Fig. 3(A), the aspirate-guiding tube 110 according to the present embodiment includes the guiding tube body 111, an inner lid 114, a hinge mechanism 115, an end cap 116, and a connection code 117, and these are integrally molded. That is, as for the aspirate-guiding tube 110, these components are integrally molded by injection-molding thermoplastic resin such as polyethylene (PE), polypropylene (PP), polyvinyl chloride (PVC), ABS resin, or acrylic resin (PMMA).

As for the guiding tube body 111, the connection tube 112 that corresponds to the suction connection portion that is connected to the suction tube is provided on the surface. The diameter of the connection tube 112 decreases in a direction toward an end, and the end corresponds to an opening portion 112a. The inner diameter of the opening portion 112a is, for example, about 5 mm. The shape of the connection tube 112 is not limited to a shape illustrated in Fig. 3(A) and Fig. 3(B) such that the diameter stepwise decreases in the direction toward the end, provided that the diameter decreases in the direction toward the end.

According to the present embodiment, the "suction connection portion" that is connected to the suction tube is the connection tube 112 that is provided on the guiding tube body 111. However, the suction connection portion is not limited to the connection tube 112 that is inserted into the suction tube. That is, an example of the "suction connection portion" that is provided on the guiding tube body 111 may be a hole that enables the suction tube to be inserted and fixed in the surface of the guiding tube body 111.

As illustrated in Fig. 3(A), a holder 113 that can grip a tubular member is provided at an edge of the outer circumference of the lower end of the guiding tube body 111. As for the holder 113, a pair of grip portions 113a protrude from the edge of the outer circumference of the lower end of the guiding tube body 111. The holder 113 can grip the tubular member, an example of which is the end of the suction tube of the suction apparatus with the end directed upward and fixed. As for the holder 113, as illustrated in Fig. 3(A), an inner circumferential surface has a diameter that increases from a base end toward an end and is an inner tapered surface.

As illustrated in Fig. 3(B), an opening portion 111a that has an inner diameter of, for example, about 17 mm is provided at a portion of the guiding tube body 111 that faces and is opposite the connection tube 112, an inner circumferential surface 111b thereof is a curved surface that continuously extends toward a base end of the connection tube 112. The inner lid 114 that is capable of opening and closing by using the hinge mechanism 115 with respect to the opening portion 111a is provided near the opening portion 111a of the guiding tube body 111. The inner lid 114 includes an opening portion 114a that has an inner diameter of, for example, about 5 mm at an end. The opening portion 114a is capable of opening and closing by using the end cap 116 that is connected to an end of the inner lid 114 with the connection code 117 interposed therebetween.

The inner lid 114 functions as a so-called olive tube and is used during nostril suctioning. The inner lid 114 can be also used for vacuuming in a manner in which the inner lid 114 closes with respect to the opening portion 111a of the guiding tube body 111, and the end of the inner lid 114 is subsequently brought into close contact with the engagement projection portion 101a of the aspirate outlet 101 that is included in the connection source switch 105. That is, the aspirate-guiding tube 110 can be also used so as to remove the sputum that is lifted up to the upper portion of the respiratory tract by using the suction catheter 107 after vacuuming with the inner lid 114 closed with respect to the opening portion 111a. The aspirate-guiding tube 110 according to the present embodiment includes the inner lid 114 and the end cap 130 but is not limited thereto. That is, the aspirate-guiding tube 110 can be used for the aspirate-guiding apparatus 100, provided that at least the guiding tube body 111 that includes the connection tube 112 that is connectable to the suction tube of the suction apparatus is provided.

As for the aspirate-guiding tube 110 according to the present embodiment, the connection tube 112 is connected to the suction tube of the suction apparatus, and the suction force (negative pressure) from the suction apparatus is consequently transmitted to the opening portion 114a via the connection tube 112. For this reason, the end portion 107a of the suction catheter 107 that is inserted into the connection tube 112 of the aspirate-guiding tube 110 is inserted into the aspirate outlet 101 of the connection source switch 105, and the suction force (negative pressure) from the suction apparatus is consequently transmitted to the end portion 107a of the suction catheter 107.

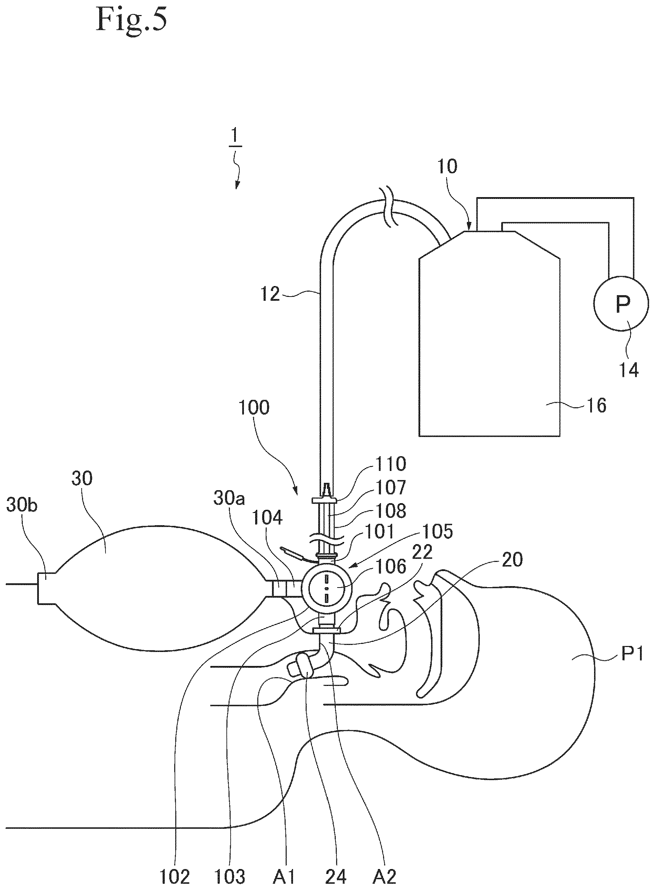

The switching valve 105a of the connection source switch 105 thus connects the aspirate outlet 101 and the patient connection port 103 to each other. This enables the aspirate such as sputum in the lower respiratory tract that extends from the trachea to the terminal bronchioles in the lung to be moved to the upper respiratory tract by vacuuming (guidance). The aspirate such as sputum that is guided to the upper respiratory tract by vacuuming can be continuously suctioned as it is. In the case where the suction catheter 107 and the cover member 108 are directly connected to a suction tube 12 (see Fig. 5 to Fig. 7) of a suction apparatus 10, the aspirate-guiding tube 110 can be omitted.

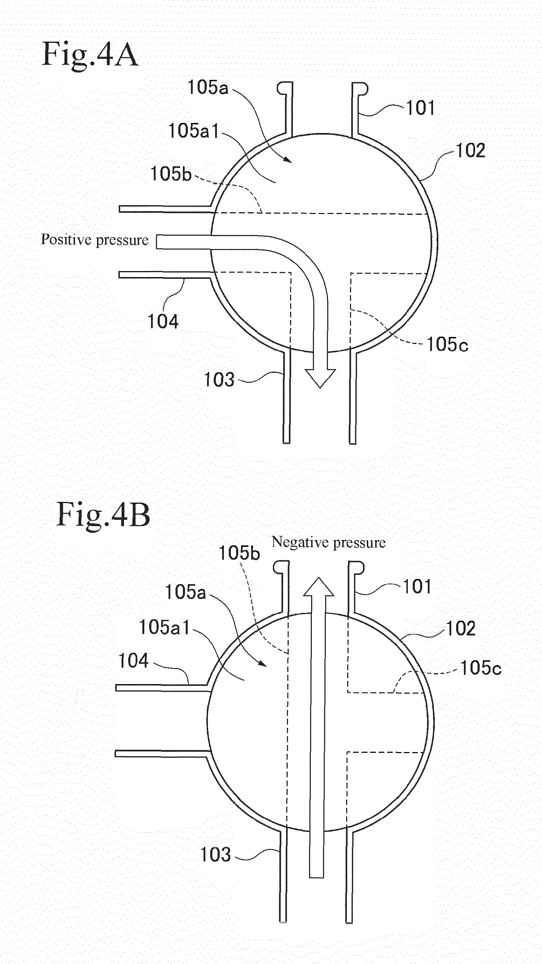

The motion of the switching valve of the aspirate-guiding apparatus according to an embodiment of the present disclosure to switch the connection source of the patient connection port 103 will now be described with reference to the drawings. Fig. 4(A) and Fig. 4(B) illustrate the motion of the switching valve of the aspirate-guiding apparatus according to an embodiment of the present disclosure to switch the connection source of the patient connection port.

As for the aspirate-guiding apparatus 100 according to the present embodiment, the aspirate-guiding tube 110 and the aspirate outlet 101 of the connection source switch 105 are connected to each other with the suction catheter 107 and the cover member 108 interposed therebetween. The aspirate-guiding tube 110 is mounted at the end of the suction tube of the suction apparatus. The connection source switch 105 includes the switching valve 105a. The switching valve 105a is capable of switching the connection source of the patient connection port 103 to the aspirate outlet 101 or the intake port 104.

For this reason, in the case where the lung is inflated by ventilation under positive pressure (referred to below as the "positive pressure ventilation") by using the intake apparatus before the aspirate such as sputum is suctioned from the lower respiratory tract of the patient, as illustrated in Fig. 4(A), the switching valve 105a directs the suction ventilation path 105b in the horizontal direction. The switching valve 105a directs the suction ventilation path 105b in the horizontal direction, and consequently, the suction ventilation path 105b can be in communication with the intake port 104. At the same time, as for the switching valve 105a, the intake ventilation path 105c is directed downward in the vertical direction and can be in communication with the patient connection port 103. That is, as for the switching valve 105a according to the present embodiment, the suction ventilation path 105b is in communication with the intake port 104 during the positive pressure ventilation, and the valve member 105a1 is disposed in the casing 102 such that the intake ventilation path 105c is in communication with the patient connection port 103.

As for the switching valve 105a, the valve member 105a1 is thus disposed, and consequently, an end of the suction ventilation path 105b that is directed in the horizontal direction can be in communication with the intake port 104, and at the same time, the intake ventilation path 105c is directed downward in the vertical direction and can be in communication with the patient connection port 103. At this time, the other end of the suction ventilation path 105b faces the casing 102 and is closed as illustrated in Fig. 4(A). At the same time, the aspirate outlet 101 into which the end portion 107a (see Fig. 1) of the suction catheter 107 is inserted is closed by a portion of the valve member 105a1 at which the intake ventilation path 105c is not provided.

The suction force (negative pressure) from the suction apparatus is not transmitted to the patient connection port 103 with the aspirate outlet 101 closed by the portion of the valve member 105a1 at which the intake ventilation path 105c is not provided. At the same time, the intake port 104 is in communication with the patient connection port 103. For this reason, air that is fed from the intake port 104 that is connected to the intake apparatus flows into an end portion of the suction ventilation path 105b, subsequently passes through the intake ventilation path 105c, and is fed to the patient connection port 103. In this way, the air that is fed from the intake port 104 that is connected to the intake apparatus is thus fed to the lung of the patient via the patient connection port 103, and the positive pressure ventilation is performed.

In the case where the aspirate such as sputum in the respiratory tract to be suctioned is suctioned after the positive pressure ventilation ends, as illustrated in Fig. 4(B), the switching valve 105a is rotated in the right-hand direction until the suction ventilation path 105b connects the aspirate outlet 101 and the patient connection port 103 to each other. After the switching valve 105a thus starts to rotate, the suction force (negative pressure) from the suction apparatus is transmitted to the suction ventilation path 105b. At this time, the suction ventilation path 105b is directed in the vertical direction, and the intake ventilation path 105c faces the casing 102 and is closed when the aspirate outlet 101 and the patient connection port 103 are connected to each other.

Subsequently, the end portion 107a of the suction catheter 107 is inserted toward the patient connection port 103 from the aspirate outlet 101 so as to extend through the suction ventilation path 105b. As for the suction catheter 107, the base end portion 107b is connected to the connection tube 112 of the aspirate-guiding tube 110. The connection tube 112 of the aspirate-guiding tube 110 is connected to the suction tube of the suction apparatus. For this reason, the suction force (negative pressure) from the suction apparatus is transmitted from the trachea of the patient to the lung via the end portion 107a of the suction catheter 107. Consequently, vacuuming (guidance) for moving the aspirate such as sputum in the lower respiratory tract to the upper respiratory tract is efficiently performed by using the suction force (negative pressure) from the suction apparatus. The aspirate such as sputum that is moved by vacuuming to the upper respiratory tract is efficiently suctioned by the suction catheter 107 as it is and is removed.

In the case where vacuuming, suctioning, and removing the aspirate by using the suction catheter 107 that is inserted into the patient connection port 103 are ended, the suction catheter 107 that is inserted is returned to the aspirate outlet 101, and the operation to rotate the switching valve 105a is stopped. The operation to rotate the switching valve 105a is thus stopped, and consequently, the suction ventilation path 105b of the switching valve 105a is directed in the horizontal direction in the original neutral state by using the restoring force of the flat spiral spring 109. For this reason, the portion of the valve member 105a1 at which the intake ventilation path 105c is not provided closes the aspirate outlet 101 from the inner circumferential surface of the casing 102 again, and the negative pressure from the suction apparatus is blocked.

According to the present embodiment, the operation of the operation unit 106 to switch the switching valve 105a can thus easily switch the connection source of the patient connection port 103 of the connection source switch 105 to the aspirate outlet 101 or the intake port 104. That is, the negative pressure from the suction apparatus 10 is transmitted to the patient connection port 103 only while the operation unit 106 performs the process to rotate the switching valve 105a. For this reason, vacuuming is easily performed, and the aspirate can be subsequently suctioned and removed.

An aspirate-suctioning system and a method of guiding the aspirate by using the aspirate-guiding apparatus 100 according to an embodiment of the present disclosure will now be described with reference to the drawings. Fig. 5 illustrates a schematic structure of the aspirate-suctioning system according to an embodiment that uses the aspirate-guiding apparatus according to an embodiment of the present disclosure. In the method of guiding the aspirate by using an aspirate-suctioning system 1 according to an embodiment illustrated in Fig. 5, sputum as the aspirate is guided by using a tracheal cannula (a wearable ventilation member) that is worn on a patient during trachea incision and is suctioned.

The aspirate-suctioning system 1 is used when the aspirate an example of which is a biological fluid such as sputum that is clogged in, for example, the lung not illustrated or trachea A1 that is the respiratory tract of a patient P1 that is a living body is suctioned. As for the aspirate-suctioning system 1, as illustrated in Fig. 5, the aspirate-guiding tube 110 and the aspirate outlet 101 of the connection source switch 105 that includes the switching valve 105a are connected to each other by using the suction catheter 107 and the cover member 108. At this time, the connection tube 112 of the aspirate-guiding tube 110 is connected to an end of the suction tube 12 of the suction apparatus 10. As for the aspirate-suctioning system 1 according to the present embodiment, as illustrated in Fig. 5, the intake port 104 of the connection source switch 105 is connected to an elastic bag 30.

The suction apparatus 10 includes a suction pump 14 that corresponds to a negative pressure source that applies suction pressure in the suction tube 12 composed of, for example, flexible resin such as polyurethane and a container portion 16 that contains the aspirate that is suctioned from the suction tube 12. The suction apparatus 10 has a function of suctioning the aspirate. In the case where a potable suction device is used as the suction apparatus 10, the aspirate-guiding tube 110 according to the present embodiment can be directly connected to the portable suction device for use. For this reason, the suction tube 12 may be omitted in this case. As for the aspirate-suctioning system 1 according to the present embodiment, the aspirate-guiding tube 110 is mounted at the end of the suction tube 12, but the aspirate-guiding tube 110 may be integrated with the suction tube 12.

The elastic bag 30 is a member that is composed of an elastic body such as silicone and that has a bag shape such as an elliptical sphere and functions as an intake apparatus that enables manual ventilation. The elastic bag 30 has an end that has a discharge port 30a through which air in the elastic bag 30 is discharged to the outside and another end that has an outdoor air introduction port 30b that has a backflow prevention function and through which outdoor air is introduced inward in a direction. The elastic bag 30 that is thus configured is manually compressed and restored, and consequently, the positive pressure ventilation can be performed.

As for The aspirate-suctioning system 1 according to the present embodiment, the intake apparatus that is connected to the intake port 104 is not limited to the elastic bag 30 such as a bag valve mask that manually assists ventilation. For example, an inelastic bag such as a Jackson Rees bag may be used as the intake apparatus. The intake apparatus can be an invasive positive pressure intake apparatus artificial respirator for, for example, tracheal intubation or a mechanical intake apparatus such as a noninvasive mask artificial respirator such as NPPV (Noninvasive Positive Pressure Ventilation).

According to the present embodiment, as illustrated in, for example, Fig. 5, the patient connection port 103 of the connection source switch 105 is inserted into an incision hole A2 that is formed in the throat of the patient P1 and is connected to a tracheal cannula 20 that is fixed by using a fixing plate 22 and a cuff 24. At this time, as for the aspirate-guiding tube 110, the suction tube 12 of the suction apparatus 10 is connected to the connection tube 112, and the suction catheter 107 and the cover member 108 are connected to the opening portion. The elastic bag 30 is connected to the intake port 104 of the connection source switch 105. The patient connection port 103 of the connection source switch 105 is connected to an end 20a of the tracheal cannula 20. Subsequently, the positive pressure ventilation is started by using the elastic bag 30 for an improvement in atelectasis and the oxygenation of the lung of the patient.

After the positive pressure ventilation ends, the operation unit 106 performs the operation to rotate the switching valve 105a, and consequently, the connection source of the patient connection port 103 is switched from the intake port 104 to the aspirate outlet 101 of the connection source switch 105. The suction catheter 107 that is mounted on the connection tube 112 of the aspirate-guiding tube 110 is inserted from the aspirate outlet 101, and a suctioning operation of the suction apparatus 10 is started. The suctioning operation is started, and the suction force (negative pressure) from the suction apparatus 10 is consequently transmitted to the tracheal cannula 20 via the aspirate-guiding tube 110 and the suction catheter 107. The negative pressure from the suction apparatus 10 is transmitted to the tracheal cannula 20, and vacuuming (guidance) for moving the aspirate such as sputum in the lower respiratory tract to the upper respiratory tract is consequently performed.

That is, as for the aspirate-suctioning system 1 and the method of guiding the aspirate according to the present embodiment, the patient connection port 103 of the connection source switch 105 is connected to the end 20a of the tracheal cannula 20. As for the connection source switch 105, the intake port 104 is connected to the elastic bag 30. The patient connection port 103 is thus connected to the end 20a of the tracheal cannula 20, and the lung of the patient P1 is ventilated by the positive pressure ventilation by using positive pressure from the elastic bag 30. After the positive pressure ventilation ends, the operation unit 106 performs the operation to rotate the switching valve 105a, and the connection source of the patient connection port 103 is switched from the intake port 104 to the aspirate outlet 101.

The operation unit 106 performs the operation to switch the connection source of the patient connection port 103 by using the switching valve 105a, the suction ventilation path 105b of the switching valve 105a is consequently directed in the vertical direction, and the aspirate outlet 101 and the patient connection port 103 are in communication with each other. At this time, the aspirate-guiding tube 110 is connected to the suction tube 12. The aspirate-guiding tube 110 is connected to the aspirate outlet 101 of the connection source switch 105 with the suction catheter 107 and the cover member 108 interposed therebetween. As for the connection source switch 105, the patient connection port 103 is connected to the end 20a of the tracheal cannula 20. For this reason, the aspirate such as sputum is guided to the upper respiratory tract via the tracheal cannula 20 by using the suction force (negative pressure) from the suction apparatus 10. Subsequently, the aspirate such as sputum in the trachea A1 that is guided to the upper respiratory tract is suctioned by the suction catheter 107.

After suctioning the aspirate such as sputum in the trachea A1 ends, the suction catheter 107 is extracted from the aspirate outlet 101. The operation of the operation unit 106 to rotate the switching valve 105a is stopped to restore the direction of the suction ventilation path 105b from the vertical direction to the horizontal direction. The operation to rotate the switching valve 105a is stopped, and the state of the switching valve 105a consequently returns to a neutral state in which the suction ventilation path 105b is directed in the horizontal direction by using the restoring force of the flat spiral spring 109 (see Fig. 2(D)).

Consequently, the connection source of the patient connection port 103 is switched from the aspirate outlet 101 that is inserted into the suction catheter 107 to the intake port 104 that is connected to the elastic bag 30. As a result, the positive pressure ventilation is performed again from the elastic bag 30, and the state of the lung returns to a normal state in which air is present. In this way, as for the aspirate-suctioning system 1 according to the present embodiment, the operation to switch the switching valve 105a of the connection source switch 105 facilitates an operation to switch to the positive pressure ventilation that is performed before and after a suction treatment that is performed by vacuuming under negative pressure.

As for the aspirate-suctioning system 1 and the method of guiding the aspirate according to the present embodiment, the aspirate-guiding apparatus 100 first performs vacuuming for moving, to the upper respiratory tract, the aspirate such as sputum in the lower respiratory tract that extends from the trachea A1 to the terminal bronchioles. After vacuuming, the suction catheter 107 suctions the aspirate that is moved in the trachea A1 toward the upper respiratory tract. That is, the aspirate-guiding apparatus 100 of the aspirate-suctioning system 1 according to the present embodiment guides the aspirate to a portion of the trachea A1 near the upper respiratory tract in order to make the aspirate such as sputum in the lower respiratory tract likely to be suctioned by using a suction instrument such as the suction catheter 107.

The aspirate-suctioning system 1 suctions and removes the aspirate that is guided to the upper respiratory tract by using the aspirate-guiding apparatus 100. The aspirate-suctioning system 1 according to the present embodiment is also used to switch to the positive pressure ventilation that is performed before and after vacuuming by performing the operation to switch the switching valve 105a of the connection source switch 105. That is, the aspirate-suctioning system 1 is used to easily switch to the positive pressure ventilation with the patient connection port 103 connected to the end 20a of the tracheal cannula 20.

A sputum suctioning method that can be performed by using the aspirate-suctioning system 1 will now be described as an example of the method of guiding the aspirate according to the present embodiment. When the aspirate such as sputum is suctioned from the lower respiratory tract of the patient P1, the lung of the patient P1, for example, is auscultated by using a stethoscope, and the lung is subsequently inflated by the positive pressure ventilation for 5 seconds under a positive pressure of 40 cmH₂O by using the intake apparatus such as an elastic bag. At this time, if a peripheral portion away from a position at which the aspirate is stored is atelectasis, it is difficult to lift the aspirate up to the upper respiratory tract even by vacuuming in some cases because there is no air at the lung periphery. For this reason, the positive pressure ventilation is performed before the suction treatment during the sputum suctioning by using the aspirate-suctioning system 1.

In many cases where the sputum suctioning is needed, the state of the respiration of the patient P1 is bad, and blood oxygen saturation is low. Accordingly, the patient P1 cannot breathe, and hypoxia is likely to occur during the sputum suctioning. In view of this, according to the present embodiment, the positive pressure ventilation is performed with oxygen mixed, the condition of the patient is stabilized, and the suction treatment is subsequently started in the case where the positive pressure ventilation described above is performed before the suction treatment. The positive pressure ventilation may be performed with high concentration of oxygen mixed in order to stabilize the condition of the patient P1 in a short time. If the positive pressure ventilation does not need to be performed in a short time, however, the positive pressure ventilation may be performed by using normal air containing oxygen. A pressure ventilator (an intake apparatus) such as an elastic bag can be included in the aspirate-suctioning system 1 according to the present embodiment.

Subsequently, vacuuming is performed, for example, under a negative pressure of 60 cmH₂O, that is, a pressure of -60 cmH₂O for 5 seconds, the entire lung is contracted, and the aspirate such as sputum is guided to the upper respiratory tract. The aspirate such as sputum that is guided to the upper respiratory tract is suctioned by the suction instrument such as a suction catheter. At this time, the lung is contracted by vacuuming, and atelectasis subsequently occurs. Accordingly, the positive pressure ventilation is performed again under a positive pressure of 40 cmH₂O for 5 seconds, and the state of the lung consequently returns to a normal state in which air is present. The aspirate-suctioning system 1 according to the present embodiment thus performs the positive pressure ventilation before and after a suction treatment in which the entire lung is contracted by vacuuming under negative pressure.

In the method of guiding the aspirate by using the aspirate-suctioning system 1 according to the present embodiment, the patient P1 is auscultated, a cycle of the positive pressure ventilation, vacuuming under negative pressure, suctioning the aspirate that is guided to the upper respiratory tract, and subsequently performing the positive pressure ventilation again is repeated until the symptoms of the patient P1 are improved. The aspirate-suctioning system 1 according to the present embodiment thus mounts the suction catheter 107 on the aspirate-guiding tube 110 that is connected to the end of the suction tube 12 of the suction apparatus 10 and subsequently performs a vacuuming operation to guide the aspirate in the lower respiratory tract to the upper respiratory tract. As for the aspirate-suctioning system 1 according to the present embodiment, the sputum suctioning is easily performed at a low degree of invasion by performing the suctioning operation to suction the aspirate that is guided to the upper respiratory tract by using the suction catheter 107 (the suction instrument).

According to the present embodiment, the positive pressure ventilation is performed before and after a sputum suctioning operation, the sputum suctioning is consequently performed at a low degree of invasion, and the pain of the patient P1 is reduced. In particular, according to the present embodiment, when the positive pressure ventilation is performed before and after the suctioning operation for vacuuming under negative pressure, the operation to rotate the switching valve 105a is performed with the patient connection port 103 of the connection source switch 105 connected to the end 20a of the tracheal cannula 20. The operation unit 106 thus performs the operation to rotate the switching valve 105a and can consequently easily perform the operation to switch between vacuuming under negative pressure and the positive pressure ventilation. For this reason, foreign substances such as virus and germs are inhibited from entering from the outside during the operation to switch, and closed sputum suctioning can be more hygienically performed.

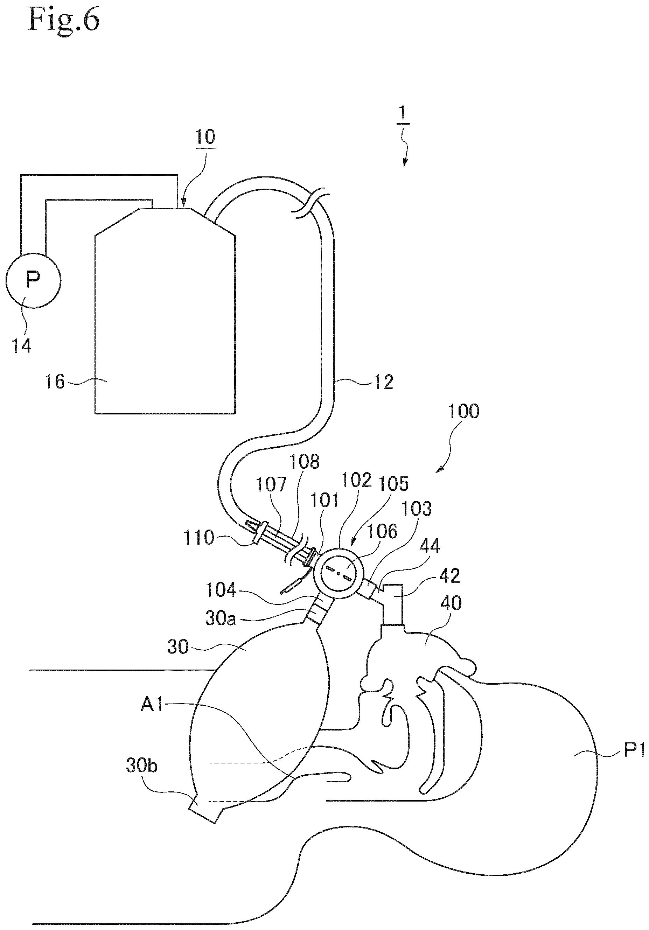

As for the aspirate-suctioning system 1 according to the present embodiment, the patient connection port 103 of the connection source switch 105 is connected to the tracheal cannula 20 that is worn at the throat of the patient P1 for vacuuming, and the aspirate that is guided to the upper respiratory tract is subsequently suctioned by the suction catheter 107. The aspirate-suctioning system 1 according to the present embodiment can be used in other aspects. That is, the structure of the aspirate-suctioning system 1 according to the present embodiment may be as illustrated in Fig. 6 even when the wearable ventilation member such as the tracheal cannula 20 is not inserted.

Fig. 6 illustrates an example in which an intake mask 40 that covers the oral cavity of the patient P1 is used, which corresponds to another embodiment for the aspirate-guiding apparatus 100, the aspirate-suctioning system 1, and the method of guiding the aspirate by using the aspirate-suctioning system 1. According to the present embodiment, the patient connection port 103 of the connection source switch 105 is connected to a branch port 44 that branches from a connection member 42 that is installed at an end portion of the intake mask 40. As for the connection source switch 105, the elastic bag 30 is connected to the intake port 104. The aspirate-guiding tube 110 that is mounted on the suction tube 12 of the suction apparatus 10 is connected to the aspirate outlet 101 of the connection source switch 105 with the suction catheter 107 and the cover member 108 interposed therebetween, and the aspirate is guided. An example of the intake mask 40 can be an oronasal mask.

According to the present embodiment, the patient connection port 103 of the connection source switch 105 is connected to the branch port 44 of the connection member 42 of the intake mask 40, and the negative pressure from the suction apparatus 10 is consequently transmitted to the intake mask 40. The negative pressure from the suction apparatus 10 is transmitted to the intake mask 40, and the aspirate such as sputum in the lower respiratory tract that extends from the trachea A1 to the terminal bronchioles is consequently guided to the upper respiratory tract. Vacuuming the aspirate in this way enables the aspirate to be guided into the oral cavity of the upper respiratory tract. For this reason, the aspirate that is guided into the oral cavity of the upper respiratory tract can be easily suctioned by the suction instrument such as the suction catheter 107. According to the present embodiment, vacuuming is performed by using the intake mask 40, and consequently, the aspirate in the nasal cavity can be guided to the intake mask 40 for suctioning in addition to the aspirate in the oral cavity.

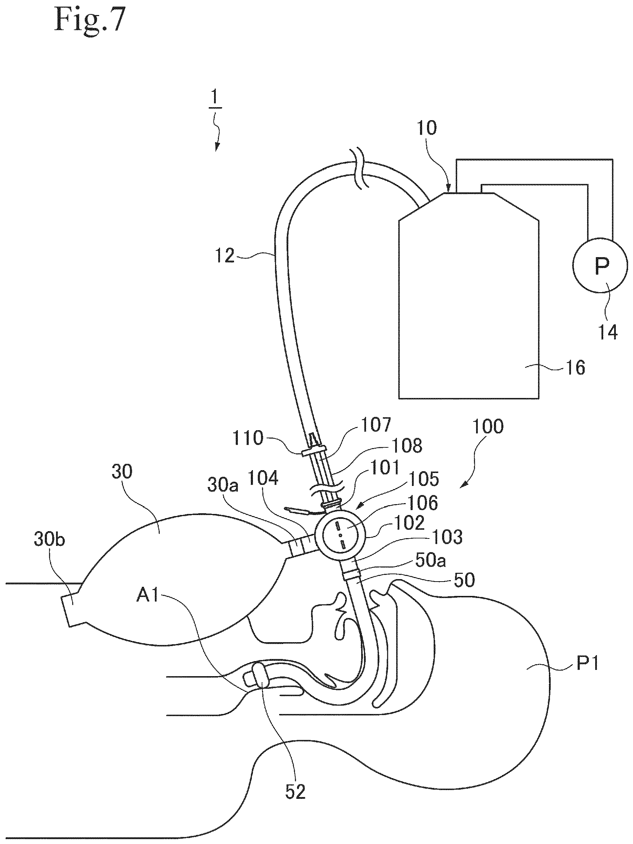

The structure of the aspirate-suctioning system 1 according to the present embodiment may be as illustrated in Fig. 7, which corresponds to another embodiment for the aspirate-guiding apparatus 100, the aspirate-suctioning system 1, and the method of guiding the aspirate by using the aspirate-suctioning system 1. That is, as illustrated in Fig. 7, the aspirate-suctioning system 1 according to the present embodiment is used when the aspirate such as sputum is suctioned and removed from the patient P1 under artificial respirator management in which a tracheal tube 50 is inserted from the mouth of the patient P1 into the trachea A1, for example, in an ICU after surgery.

Specifically, the tracheal tube 50 is inserted toward the trachea A1, and an end of the tracheal tube 50 is fixed by using a cuff 52. The patient connection port 103 of the connection source switch 105 that is included in the aspirate-guiding apparatus 100 is connected to a joint 50a that is located at a base end of the tracheal tube 50. The patient connection port 103 is connected to the joint 50a, and the negative pressure from the suction apparatus 10 is consequently transmitted, and vacuuming for guiding the aspirate such as sputum to the upper respiratory tract is performed. The aspirate that is guided to the upper respiratory tract is suctioned by the suction catheter 107, and consequently the aspirate is removed.

The patient connection port 103 of the connection source switch 105 is thus connected to the joint 50a of the tracheal tube 50, the negative pressure from the suction apparatus 10 is consequently transmitted, and the aspirate such as sputum in the lower respiratory tract can be guided to the upper respiratory tract. For this reason, the aspirate that is guided to the upper respiratory tract can be easily suctioned as it is by using the suction catheter 107. According to the present embodiment, when the positive pressure ventilation is performed before and after the suctioning operation, the operation unit 106 performs the operation to rotate the switching valve 105a with the patient connection port 103 of the connection source switch 105 connected to the joint 50a of the tracheal tube 50. The operation to rotate the switching valve 105a is thus performed, the operation to switch between vacuuming and the positive pressure ventilation can be easily performed. Accordingly, foreign substances such as virus and germs are inhibited from entering from the outside, and the closed sputum suctioning can be more hygienically performed.

The actions and effects of the aspirate-guiding apparatus 100 and the aspirate-suctioning system 1 according to an embodiment of the present disclosure will now be described.

The aspirate-guiding apparatus 100 according to the present embodiment is connectable to the suction apparatus 10 and the intake apparatus (the elastic bag 30), and the operation to switch the connection source of the patient connection port 103 of the connection source switch 105 can be easily performed by the operation unit 106 of the switching valve 105a. The aspirate-guiding tube 110 that is connected to the suction tube 12 of the suction apparatus 10 and the connection source switch 105 that is connected to the wearable ventilation member are connected to each other with the cover member 108 interposed therebetween. For this reason, the patient connection port 103 of the connection source switch 105 is connected to the wearable ventilation member, and the operation to switch the connection source of the patient connection port 103 is subsequently performed by using the switching valve 105a. This enables the positive pressure ventilation and vacuuming to be easily switched immediately. Consequently, foreign substances such as virus and germs are inhibited from entering from the outside, and the aspirate can be more hygienically performed in a closed manner at a low degree of invasion.

That is, the patient connection port 103 of the connection source switch 105 that is connected to the suction apparatus 10 and the intake apparatus such as the elastic bag 30 is connected to the wearable ventilation member that is worn on the patient P1, and the operation unit 106 subsequently performs the operation to rotate the switching valve 105a. The operation to rotate the switching valve 105a is thus performed, and the connection source of the patient connection port 103 is consequently switched from the intake port 104 to the aspirate outlet 101. Examples of the wearable ventilation member described herein include the tracheal tube 50, the tracheal cannula 20, and the intake mask 40.

The suction catheter 107 that is mounted on the aspirate-guiding tube 110 is thus inserted with the connection source of the patient connection port 103 switched to the aspirate outlet 101. The aspirate-guiding tube 110 is connected to the suction tube 12 of the suction apparatus 10. In this state, communication with the wearable ventilation member enables the aspirate to be guided to the upper respiratory tract and to be suctioned by the suction catheter 107 as it is. For this reason, an easy manual operation enables the aspirate to be guided to the upper respiratory tract, and accordingly, it is easy that the aspirate that is guided to the upper respiratory tract can be continuously suctioned (discharged to the outside from the body) at a low degree of invasion.

Vacuuming by using the negative pressure of the suction apparatus 10 can be performed in a manner in which the suction tube 12 is brought into close contact with and connected to the wearable ventilation member such as the tracheal tube 50 or the tracheal cannula 20. As for the suction tube 12, however, when the suction tube 12 is used by being cut by scissors, a section of the suction tube 12 is nonuniform, and a tube diameter varies. For this reason, adhesion between the suction tube 12 and the wearable ventilation member such as the tracheal tube 50 or the tracheal cannula 20 is unlikely to be ensured, and the effects of vacuuming vary depending on the skill of a practitioner. Suctioning with the suction tube 12 being in direct contact with the patient or the biological fluid defeats the purpose of the suction tube 12 and is not pharmaceutically permitted.

In view of this, according to the present embodiment, the connection tube 112 is inserted into and connected to the suction tube 12, the adhesion between the suction tube 12 and the connection tube 112 is consequently ensured, and the aspirate-guiding tube 110 and the connection source switch 105 are connected to each other with the cover member 108 interposed therebetween. The patient connection port 103 of the connection source switch 105 is connected to the wearable ventilation member such as the tracheal cannula 20 or the tracheal tube 50. Accordingly, according to the present embodiment, a practitioner who is not skilled is even likely to ensure adhesion between components. For this reason, the effects of vacuuming are exerted with certainty, and anybody can perform vacuuming in the same manner.

That is, in the case where a suction catheter that is connected to a suction tube is inserted into the respiratory tract for the sputum suctioning, the effects of suctioning are typically not sufficiently exerted unless an end of the suction catheter reaches the sputum to be suctioned. If the suction catheter is blindly inserted, for example, there is a concern that the respiratory tract of the patient is damaged due to stimulation for the respiratory tract caused by the suction catheter that is deeply inserted. The mechanical insufflation exsufflation (MI-E) achieves sputum discharge at a low degree of invasion in a short time. However, the settings and operation of an apparatus that performs the mechanical insufflation exsufflation (MI-E) are difficult, and accordingly, a skilled technique for the operation of the sputum discharge by using the apparatus is needed, and the cost of the apparatus itself is high.

In contrast, as for the aspirate-guiding apparatus 100 according to the present embodiment, the aspirate-guiding tube 110 that is mounted at the end of the suction tube 12 is connected to the aspirate outlet 101 of the connection source switch 105 with the suction catheter 107 and the cover member 108 interposed therebetween. The connection source switch 105 is connected to the wearable ventilation member such as the tracheal cannula 20 or the tracheal tube 50 that is worn on the patient P1.

According to the present embodiment, the use of the aspirate-guiding tube 110 as a connection attachment in this way enables adhesion between the aspirate-guiding tube 110 and the suction tube 12 to be ensured. According to the present embodiment, the connection source switch 105 that is connected to the aspirate-guiding tube 110 with the cover member 108 interposed therebetween is connected to the wearable ventilation member. For this reason, the suction force (negative pressure) from the suction apparatus 10 does not leak from a portion at which the suction tube 12 and the connection tube 112 of the aspirate-guiding tube 110 are in close contact with each other but is transmitted to the wearable ventilation member such as the tracheal cannula 20 or the tracheal tube 50 via the cover member 108, the suction catheter 107, and the connection source switch 105.

The aspirate-guiding apparatus 100 according to the present embodiment includes the connection source switch 105 that includes the switching valve 105a that switches the connection source of the patient connection port 103. The connection source switch 105 is thus included, and the connection source switch 105 can switch a circuit only during the suctioning operation and can automatically restore an artificial respiration circuit after the suctioning operation ends. For this reason, it is not necessary to remove a respiration circuit for every suctioning operation, the risk of, for example, a fatal accident due to neglection of connection of the artificial respirator after the suctioning operation ends is reduced, and the suctioning operation can be more safely performed. Vacuuming can be performed with the aspirate-guiding tube 110 being in close contact with the engagement projection portion 101a of the aspirate outlet 101 of the connection source switch 105. For this reason, connecting the suction catheter 107 and the cover member 108 to the aspirate outlet 101 of the connection source switch 105 easily enables upgrading to a closed vacuuming system.

According to the present embodiment, the connection tube 112 of the aspirate-guiding tube 110 is connected to the end of the suction tube 12, and the patient connection port 103 of the connection source switch 105 is subsequently connected to the wearable ventilation member such as the tracheal cannula 20 or the tracheal tube 50 that is worn on the patient P1. The operation unit 106 of the switching valve 105a is rotated while being connected to the wearable ventilation member, and the connection source of the patient connection port 103 is switched to the aspirate outlet 101. The suction catheter 107 that is mounted on the aspirate-guiding tube 110 that is connected to the suction tube 12 of the suction apparatus 10 is inserted into the aspirate outlet 101. Consequently, the aspirate such as sputum at the periphery in the lower respiratory tract of the patient P1 is moved to the upper respiratory tract by vacuuming via the patient connection port 103. For this reason, the aspirate that is moved to the upper respiratory tract is easily suctioned and removed as it is by using the suction catheter 107 that is connected to the aspirate-guiding tube 110.

In particular, according to the present embodiment, the aspirate-guiding tube 110 is connected to the suction tube 12 of the suction apparatus 10, and the connection source switch 105 includes the switching valve 105a that switches the connection source of the patient connection port 103. The aspirate-guiding tube 110 and the aspirate outlet 101 of the connection source switch 105 are connected to each other by using the suction catheter 107 and the cover member 108. For this reason, the negative pressure from the suction apparatus 10 is transmitted to the suction catheter 107 that is mounted on the connection tube 112 of the aspirate-guiding tube 110.

Subsequently, the switching valve 105a is switched such that the aspirate outlet 101 and the patient connection port 103 are in communication with each other, and the suction catheter 107 is inserted. As a result, the aspirate that is guided to the upper respiratory tract can be easily suctioned and removed as it is by the suction catheter 107 after vacuuming under the negative pressure from the end portion 107a of the suction catheter 107. After suctioning and removing the aspirate end, the suction catheter 107 is extracted to the aspirate outlet 101, and the operation of the operation unit 106 to rotate the switching valve 105a is stopped. The rotating operation of the switching valve 105a is thus stopped, and consequently, the state of the switching valve 105a returns to the original neutral state, and the suctioning operation can be easily stopped.

For this reason, the cover member 108 can prevent foreign substances such as virus and germs from entering from the outside while vacuuming for guiding the aspirate such as sputum at the periphery in the lower respiratory tract of the patient P1 to the upper respiratory tract is performed and the aspirate that is guided to the upper respiratory tract is suctioned and removed. Accordingly, the aspirate can be hygienically suctioned and removed in a further closed manner. In addition, according to the present embodiment, the suction catheter 107 and the cover member 108 that connect the aspirate-guiding tube 110 and the connection source switch 105 to each other can be easily installed and removed. For this reason, it is easier that the aspirate such as sputum can be hygienically suctioned and removed in a closed manner.

According to the present embodiment, the positive pressure ventilation is performed by the intake apparatus such as the elastic bag 30 before the vacuuming operation under negative pressure and after the suctioning operation of the aspirate that is guided to the upper respiratory tract as described above. The positive pressure ventilation is thus performed, and consequently, the aspirate such as sputum can be easily suctioned and removed at a low degree of invasion. In particular, according to the present embodiment, the operation of the operation unit 106 of the switching valve 105a to switch the connection source of the patient connection port 103 of the aspirate-guiding apparatus 100 that is connected to the suction apparatus 10 and the intake apparatus can be easily performed. For this reason, foreign substances such as virus and germs are inhibited from entering from the outside, and the closed sputum suctioning can be more hygienically performed.

As for the aspirate-guiding apparatus 100 according to the present embodiment, the aspirate-guiding tube 110 is thus used as an attachment that is mounted at the end of the suction tube 12. As for the aspirate-guiding apparatus 100 according to the present embodiment, the connection source switch 105 that includes the switching valve 105a is used as an attachment that is connected to the wearable ventilation member that is worn on the patient P1 by using the patient connection port 103.

According to the present embodiment, the use of the aspirate-guiding tube 110 and the connection source switch 105 enables the aspirate to be easily guided to the upper respiratory tract. Accordingly, the aspirate that is guided to the upper respiratory tract is likely to be suctioned, and the efficiency of suctioning is improved. In particular, a method of blindly inserting the suction catheter deeply into the respiratory tract of the patient P1 and provoking a cough during the sputum suctioning gives the patient P1 pain and carries the risk of serious complication such as a cerebrovascular accident due to damage to the respiratory tract or increased blood pressure. However, the aspirate-guiding apparatus 100 according to the present embodiment can reduce the pain and the risk of complication.

According to the present embodiment, the sputum suctioning is efficiently performed without using an expensive apparatus that performs the mechanical insufflation exsufflation (Ml-E). For this reason, the pain of the patient P1, for example, is reduced at a decreased cost, and the aspirate such as sputum is suctioned at a low degree of invasion such that the QOL (Quality Of Life) of the patient P1 is improved. According to the present embodiment, the aspirate-guiding tube 110 and the connection source switch 105 that are included in the aspirate-guiding apparatus 100 have simple structures. For this reason, the aspirate-guiding apparatus 100 can be mass-produced at a lower cost than that of a sputum suctioning method (for example, a tracheal endoscope or an Intrapulmonary Percussive Ventilator) in which the mechanical insufflation exsufflation (for example, MI-E) or apparatus is used, and the time required for preparation and a cleanup can be reduced.

According to the present embodiment, the aspirate can be guided in a short time with certainty unlike a sputum suctioning method such as postural drainage or squeezing (manual respiration assistance). In particular, in the case of a long-term bedridden patient, the postural drainage and squeezing carry a fracture risk due to a position change or locally manual compression. According to the present embodiment, however, the effects of suctioning the aspirate are exerted by vacuuming regardless of the posture of the patient, and accordingly, the fracture risk due to the position change or the locally manual compression can be reduced.

### (Second Embodiment)

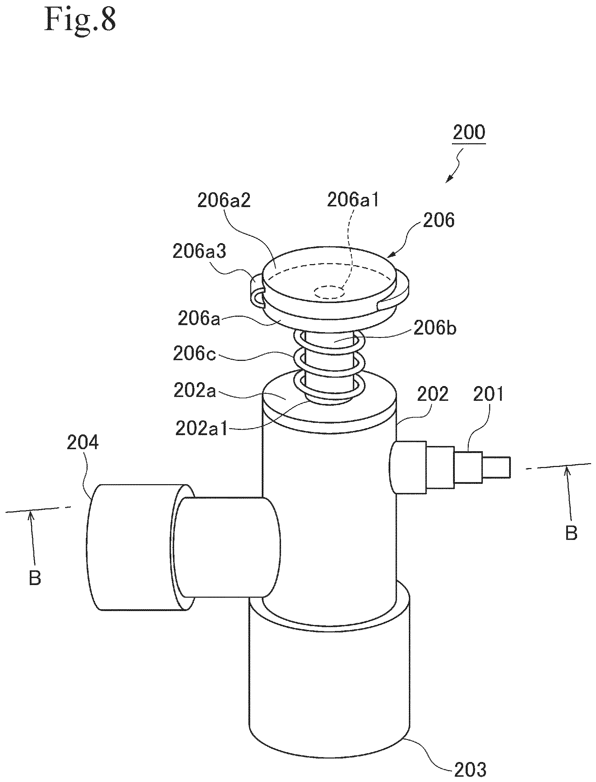

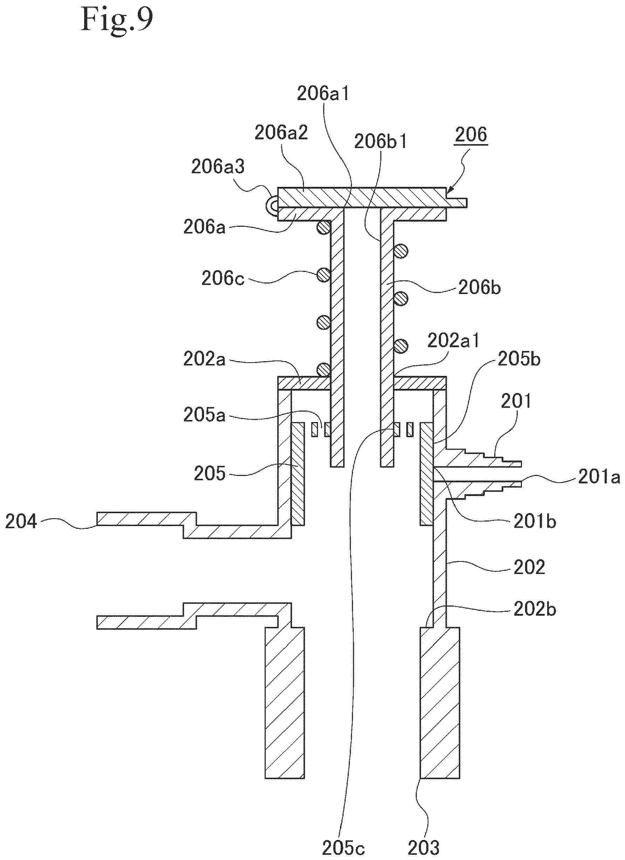

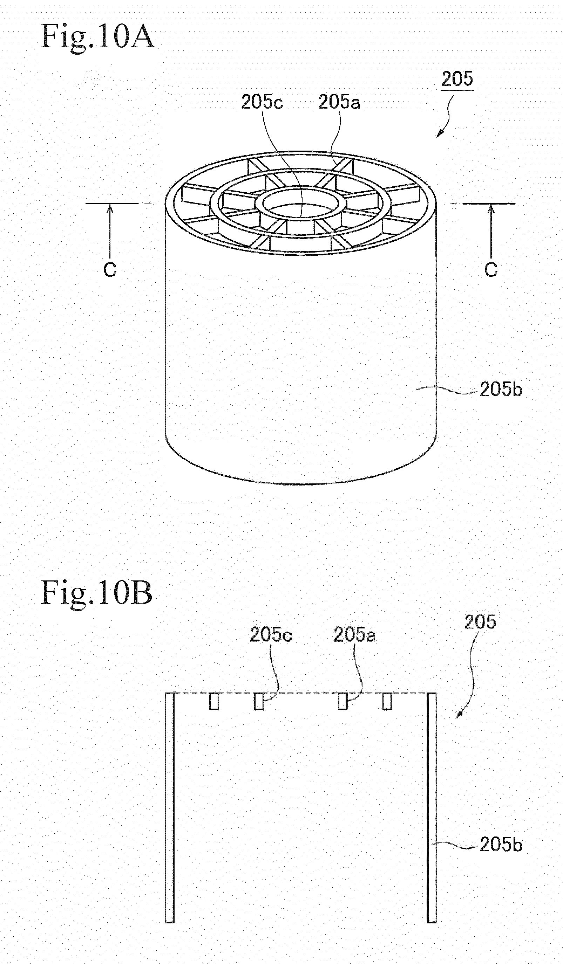

A schematic structure of an aspirate-guiding apparatus according to another embodiment of the present disclosure will now be described with reference to the drawings. Fig. 8 is a perspective view of an aspirate-guiding apparatus according to another embodiment of the present disclosure. Fig. 9 is a sectional view taken along line B-B in Fig. 8. Fig. 10(A) is a perspective view of a sliding portion that corresponds to a switching valve that is included in the aspirate-guiding apparatus according to another embodiment of the present disclosure. Fig. 10(B) is a sectional view taken along line C-C in Fig. 10(A).

An aspirate-guiding apparatus 200 according to the present embodiment is a member that is connected to the suction apparatus that is used to suction and remove the aspirate an example of which is a biological fluid such as sputum from the respiratory tract of the patient including the lower respiratory tract and the intake apparatus that is used for the positive pressure ventilation that is performed before and after the aspirate is suctioned and removed. That is, the aspirate-guiding apparatus 200 is used as a member that is mounted at the end of the suction tube connected to the suction apparatus corresponding to a stationary or portable sputum suction device and that guides the aspirate to the upper respiratory tract by using the suction force (negative pressure) of the suction apparatus. The aspirate-guiding apparatus 200 is used as a member that is mounted to a connection port of the manual or mechanical intake apparatus and that introduces air or oxygen into the lung of the patient when the positive pressure ventilation is performed before and after the aspirate is suctioned and removed.

As illustrated in Fig. 8 and Fig. 9, the aspirate-guiding apparatus 200 includes a connection portion (a connection tube) 201, a casing 202, a patient connection port 203, an intake port 204, a sliding portion 205, and an operation unit 206.

The casing 202 is a tubular member composed of resin, a closing portion 202a in which the operation unit 206 is mounted is provided at an upper end thereof, and the patient connection port 203 that has an inner diameter of, for example, about 15 mm and that is tubular is provided at a lower end thereof as illustrated in Fig. 8. The patient connection port 203 is connected to the tubular end of the wearable ventilation member such as a tracheal tube or a tracheal cannula that is worn on the patient and consequently functions as a port through which the negative pressure from the suction apparatus is transmitted to the respiratory tract of the patient. As illustrated in Fig. 9, the casing 202 includes an inner projection portion 202b at which the lower end that has the patient connection port 203 is thicker than the upper end. The inner projection portion 202b restricts a lower end of the sliding portion 205 that corresponds to the switching valve within a sliding range.

The connection tube 201 that corresponds to the connection portion that is connected to the suction tube of the suction apparatus and the intake port 204 that is connected to the connection port of the intake apparatus are provided in the side surface of the casing 202. According to the present embodiment, as illustrated in Fig. 8, the intake port 204 is provided in the side surface of the casing 202 below the connection tube 201 in the height direction of the casing 202. The arrangement of the connection tube 201 and the intake port 204 that are provided in the side surface of the casing 202 is not limited to that in aspects illustrated in Fig. 8. That is, the positions thereof in the height direction or rotational direction of the side surface of the casing 202 area "first position" and a "second position" that differ from each other.

The diameter of the connection tube 201 decreases in a direction toward an end. The end of the connection tube 201 corresponds to an opening portion 201a, and a base end corresponds to a connection hole between the suction tube and the casing 202, that is, a suction tube connection hole 201b that functions as the aspirate outlet. The inner diameter of the opening portion 201a is, for example, about 5 mm. The shape of the connection tube 201 is not limited to a shape illustrated in Fig. 8 such that the diameter stepwise decreases in the direction toward the end, provided that the diameter decreases in the direction toward the end. According to the present embodiment, the "connection portion" that is connected to the suction tube is the connection tube 201 that is provided in the side surface of the casing 202. However, the connection portion is not limited to the connection tube 201 that is inserted into the suction tube. That is, an example of the "connection portion" that is provided in the casing 202 may be a hole that enables the suction tube to be inserted and fixed in the side surface of the casing 202.

As illustrated in Fig. 8 and Fig. 9, the intake port 204 is a tubular member that is provided so as to branch in a perpendicular direction from the side surface of the casing 202. The intake port 204 functions as a port that is connected to the intake apparatus when the manual elastic bag or mechanical intake apparatus performs the positive pressure ventilation for the patient. According to the present embodiment, the intake port 204 that is connected to the intake apparatus is a tubular member that is provided in the side surface of the casing 202 so as to branch. The aspects of the intake port 204 are not limited to those of a tubular member. That is, an example of the "intake port" that is provided in the casing 202 may be a hole that is formed in the surface of the casing 202 into which the intake port of the intake apparatus can be fitted.

As for the aspirate-guiding apparatus 200 according to the present embodiment, the casing 202, the connection tube 201, and the intake port 204 are integrally molded by injection-molding thermoplastic resin. Examples of the thermoplastic resin of which the aspirate-guiding apparatus 200 is composed include polyethylene (PE), polypropylene (PP), polyvinyl chloride (PVC), ABS resin, and acrylic resin (PMMA). The aspirate-guiding apparatus 200 may be assembled in a manner in which the casing 202, the connection tube 201, and the intake port 204 are prepared as separated bodies, and for example, the connection tube 201 and the intake port 204 are subsequently stuck to the side surface of the casing 202.

As illustrated in Fig. 9, the sliding portion 205 is provided in the casing 202 so as to extend along the inner circumferential surface of the casing 202. As illustrated in Fig. 10(A) and Fig. 10(B), the sliding portion 205 is a resin member that has an opening at a lower end and an upper end surface composed of mesh where multiple air vents 205a are formed and that has a substantially cylindrical shape. An engagement hole 205c into which a shaft 206b of the operation unit 206 can be fitted is provided at the center of the upper end surface of the sliding portion 205. The sliding portion 205 functions as the switching valve that slides in the casing 202 and that closes the suction tube connection hole 201b or the intake port 204 from a position in the casing 202 by using a side surface 205b of the sliding portion 205.

The operation unit 206 has a function of performing the operation to switch the connection source of the patient connection port 203 by moving the sliding portion 205 along the casing 202. The operation unit 206 according to the present embodiment moves the sliding portion 205 along the casing 202 downward from above and consequently switches the connection source of the patient connection port 203 from the intake port 204 to the suction tube connection hole 201b. That is, before the operation unit 206 moves the sliding portion 205, the side surface 205b of the sliding portion 205 closes the suction tube connection hole 201b of the connection tube 201 such that the patient connection port 203 and the intake port 204 are in communication with each other, and air is allowed to pass therebetween. After the operation unit 206 moves the sliding portion 205 downward, the side surface 205b of the sliding portion 205 closes the intake port 204. The patient connection port 203 and the suction tube connection hole 201b are consequently in communication with each other such that air is allowed to pass therebetween via the air vents 205a of the sliding portion 205.

In the case where the arrangement of the connection tube 201 and the intake port 204 that are provided in the side surface of the casing 202 differs from that illustrated in Fig. 8, that is, in the case where the positions thereof in the height direction or rotational direction of the side surface of the casing 202 differ from the "first position" and the "second position", an operation of the operation unit 206 to slide the sliding portion 205 is changed. For example, in the case where the arrangement of the connection tube 201 and the intake port 204 in the height direction is reversed, the operation unit 206 may cause the sliding portion 205 to move downward along the casing 202 such that the side surface 205b of the sliding portion 205 closes the connection tube 201 instead of the intake port 204. In the case where the connection tube 201 and the intake port 204 are arranged differently in the direction of rotation of the side surface of the casing 202, an operation to rotate the sliding portion 205 along the side surface of the casing 202 is performed, and consequently, the side surface 205b of the sliding portion 205 may close the suction tube connection hole 201b or the intake port 204 of the connection tube 201 and may not close the other.

According to the present embodiment, the operation unit 206 causes the sliding portion 205 to move along the casing 202 and consequently switches the connection source of the patient connection port 203 to the suction tube connection hole 201b of the connection tube 201 or the intake port 204. Specifically, as illustrated in Fig. 9, the operation unit 206 includes a pressing portion 206a that is capable of being pressed from the outside, the shaft 206b that connects the pressing portion 206a and the sliding portion 205 to each other, and an elastic member 206c that is provided between the pressing portion 206a and the closing portion 202a.

As illustrated in Fig. 8, an example of the pressing portion 206a is a resin plate member that has a flange and that has, for example, a circular shape, and an opening portion 206a1 is provided at the center thereof. The opening portion 206a1 can be in communication with a through-hole 206b1 that is formed in the shaft 206b in the axial direction. The pressing portion 206a includes a lid member 206a2 that corresponds to a cap that covers the opening portion 206a1, and a connection code 206a3 that connects the lid member 206a2 and the pressing portion 206a to each other. The opening portion 206a1 is capable of opening and closing by using the lid member 206a2.

The shaft 206b is a resin tubular member that has the through-hole 206b1 in the axial direction. For this reason, after the lid member 206a2 of the pressing portion 206a is opened, the suction instrument such as a suction catheter can be inserted from the opening portion 206a1 so as to extend through the through-hole 206b1. The shaft 206b is fitted into the engagement hole 205c that is formed in the upper surface of the sliding portion 205 and consequently connects the pressing portion 206a and the sliding portion 205 to each other. The shaft 206b is provided near the upper end of the casing 202 so as to be slidable on an opening portion 202a1 of the closing portion 202a. For this reason, the sliding portion 205 that is connected to the pressing portion 206a with the shaft 206b interposed therebetween can move along the casing 202 due to movement that involves pressing the pressing portion 206a. The vicinity of the shaft 206b is preferably covered by, for example, a resin sheet in order to prevent a stain due to, for example, adhering sputum.

As illustrated in Fig. 8 and Fig. 9, the elastic member 206c is wound around the shaft 206b and is provided between the pressing portion 206a and the closing portion 202a. According to the present embodiment, the elastic member 206c includes a coil spring that includes a compression spring that has restoring force in a direction opposite a direction in which the pressing portion 206a is pressed. According to the present embodiment, the elastic member 206c includes the coil spring but may have another structure, provided that the elastic member 206c has the restoring force in the direction opposite the direction in which the pressing portion 206a is pressed. For example, a tension spring may be wound, as the elastic member, around the shaft between the closing portion 202a and the sliding portion 205 so as to have the restoring force in the direction opposite the direction in which the pressing portion 206a is pressed.

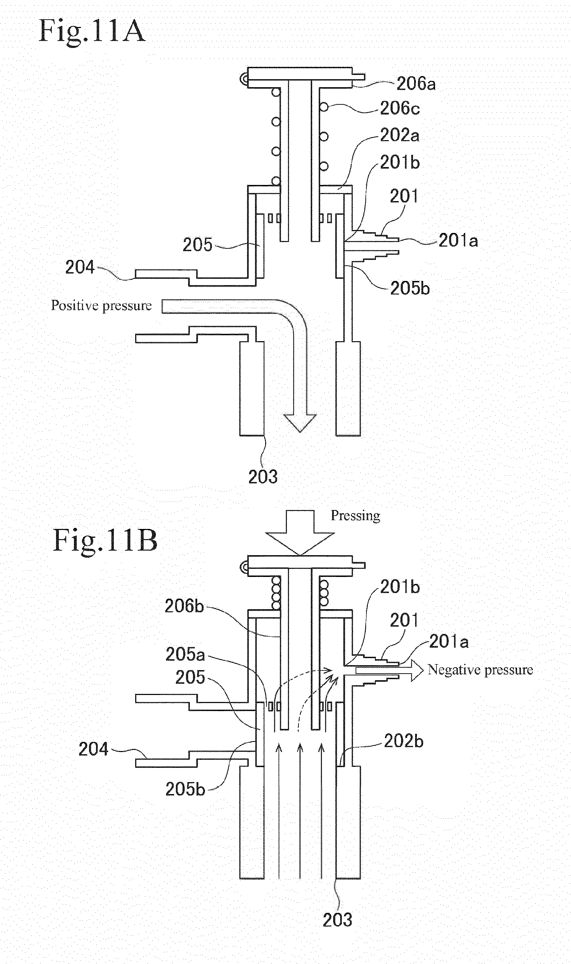

The motion of an aspirate-guiding tube according to another embodiment of the present disclosure will now be described with reference to the drawings. Fig. 11(A) and Fig. 11(B) illustrate the motion of an aspirate-guiding tube according to another embodiment of the present disclosure.

The aspirate-guiding apparatus 200 according to the present embodiment includes the patient connection port 203 at the lower end of the casing 202. The connection tube 201 that is connected to the suction tube of the suction apparatus and the intake port 204 that is connected to the intake apparatus are mounted in the side surface of the casing 202 so as to branch. An operation of the operation unit 206 to slide the sliding portion 205 can switch the connection source of the patient connection port 203 to the suction tube connection hole 201b of the connection tube 201 or the intake port 204.

For example, in the case where the intake apparatus performs the positive pressure ventilation, and the lung is inflated before the aspirate such as sputum is suctioned from the lower respiratory tract of the patient, the patient connection port 203 and the intake port 204 are in communication with each other such that air is allowed to pass therebetween. Specifically, as for the aspirate-guiding apparatus 200, as illustrated in Fig. 11(A), the side surface 205b of the sliding portion 205 closes the suction tube connection hole 201b of the connection tube 201 from a position in the casing 202. The side surface 205b of the sliding portion 205 does not close the intake port 204 from the position in the casing 202, and accordingly, the patient connection port 203 and the intake port 204 can be in communication with each other such that air is allowed to pass therebetween.

At this time, the pressing portion 206a of the operation unit 206 is not pressed, and accordingly, the state of the elastic member 206c that includes the coil spring is a neutral state. For this reason, the elastic member 206c in the neutral state causes the pressing portion 206a of the operation unit 206 to be separated from the closing portion 202a. Along with this, as illustrated in Fig. 11(A), the sliding portion 205 remains stationary near the top of the casing 202. For this reason, the side surface 205b of the sliding portion 205 closes the suction tube connection hole 201b of the connection tube 201 from the position in the casing 202.

The side surface 205b of the sliding portion 205 thus closes the suction tube connection hole 201b, and consequently, the suction force (negative pressure) from the suction apparatus is not transmitted into the casing 202. In addition, the intake port 204 and the patient connection port 203 are in communication with each other. For this reason, air that is fed from the intake port 204 that is connected to the intake apparatus is fed to the lung of the patient via the patient connection port 203, and the positive pressure ventilation is performed.

In the case where the aspirate to be suctioned such as sputum in the respiratory tract is suctioned after the positive pressure ventilation ends, as illustrated in Fig. 11(B), the pressing portion 206a is pressed. The pressing portion 206a is pressed, and consequently, the sliding portion 205 is moved downward along the casing 202 until being engaged with the inner projection portion 202b. The sliding portion 205 is thus moved downward, and consequently, the side surface 205b of the sliding portion 205 that closes the suction tube connection hole 201b of the connection tube 201 shifts from the suction tube connection hole 201b. The side surface 205b of the sliding portion 205 shifts, and consequently, the side surface 205b can close the intake port 204 from the inner circumferential surface of the casing 202 after the suction tube connection hole 201b opens.

For this reason, the suction force (negative pressure) from the connection tube 201 that is connected to the suction apparatus is transmitted from the trachea of the patient to the lung via the patient connection port 203. Consequently, vacuuming (guidance) for moving, to the upper respiratory tract, the aspirate such as sputum in the lower respiratory tract that extends from the trachea to the terminal bronchioles in the lung by using the suction force (negative pressure) from the suction apparatus. The aspirate is moved to the upper respiratory tract, the lid member 206a2 of the pressing portion 206a is subsequently opened, and the suction instrument such as a suction catheter is inserted from the opening portion 206a1 into the through-hole 206b1 of the shaft 206b. The suction instrument is thus inserted, and consequently, the aspirate such as sputum that is guided to the upper respiratory tract can be efficiently suctioned by the suction instrument.

In the case where vacuuming via the patient connection port 203 is ended, an operation to press the pressing portion 206a is stopped, and consequently, the state of the pressing portion 206a returns to the original neutral state in which the pressing portion 206a is separated from the closing portion 202a by using the restoring force of the elastic member 206c. For this reason, the sliding portion 205 moves upward along the casing 202 along with this, and the side surface 205b of the sliding portion 205 closes the suction tube connection hole 201b of the connection tube 201 from the inner circumferential surface of the casing 202 again.

According to the present embodiment, the operation of the operation unit 206 to slide the sliding portion 205 thus easily enables the connection source of the patient connection port 203 of the aspirate-guiding apparatus 200 to be switched to the suction tube connection hole 201b of the connection tube 201 or the intake port 204. That is, the operation to switch is performed such that the negative pressure from the suction apparatus is transmitted to the patient connection port 203 only while the pressing portion 206a of the operation unit 206 is pressed, and vacuuming can be easily performed.

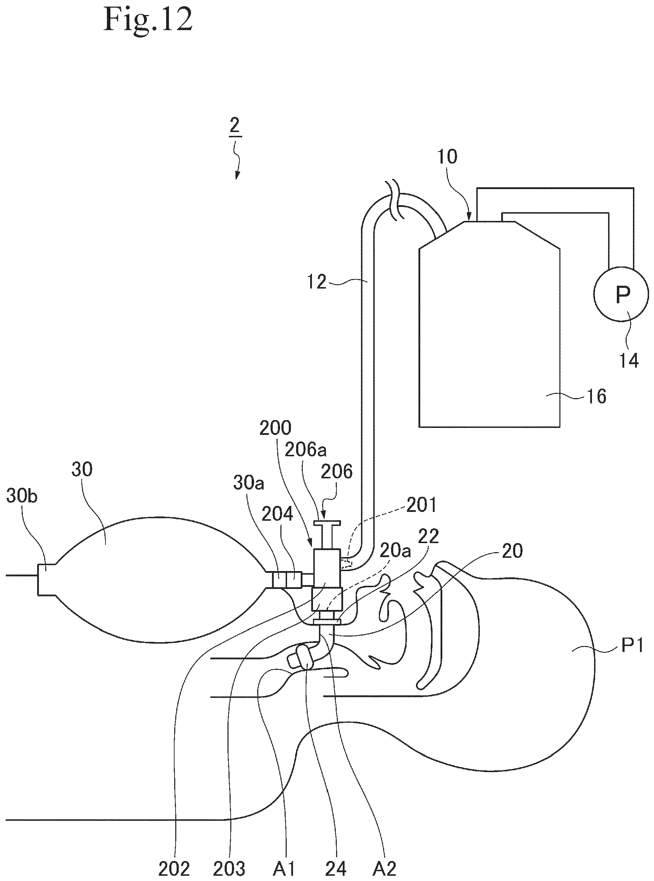

An aspirate-suctioning system and a method of guiding the aspirate that use the aspirate-guiding apparatus 200 according to another embodiment of the present disclosure will now be described with reference to the drawings. Fig. 12 illustrates a schematic structure of an aspirate-suctioning system according to an embodiment that uses the aspirate-guiding apparatus according to another embodiment of the present disclosure. Fig. 12 illustrates a situation in which sputum (the aspirate) is guided and is subsequently suctioned via the tracheal cannula (the wearable ventilation member) that is worn on the patient during the trachea incision, which corresponds to an embodiment for the aspirate-guiding apparatus 200, an aspirate-suctioning system 2 and a method of guiding the aspirate that uses the aspirate-suctioning system 2.

The aspirate-suctioning system 2 is used when the aspirate an example of which is a biological fluid such as sputum that is clogged in, for example, the lung not illustrated or the trachea A1 that is the respiratory tract of the patient P1 that is a living body is suctioned. As for the aspirate-suctioning system 2, as illustrated in Fig. 12, the connection tube 201 of the aspirate-guiding apparatus 200 described above is connected to the end of the suction tube 12 of the suction apparatus 10. As for the aspirate-suctioning system 2 according to the present embodiment, as illustrated in Fig. 12, the intake port 204 of the aspirate-guiding apparatus 200 described above is connected to the elastic bag 30 that corresponds to the intake apparatus and that is manual.

The suction apparatus 10 includes the suction pump 14 that corresponds to the negative pressure source that applies suction pressure in the suction tube 12 composed of, for example, flexible resin such as polyurethane and the container portion 16 that contains the aspirate that is suctioned from the suction tube 12. The suction apparatus 10 has the function of suctioning the aspirate. In the case where a potable suction device is used as the suction apparatus 10, the aspirate-guiding apparatus 200 according to the present embodiment can be directly connected to the portable suction device for use. For this reason, the suction tube 12 may be omitted in this case. As for the aspirate-suctioning system 2 according to the present embodiment, the aspirate-guiding apparatus 200 is mounted at the end of the suction tube 12, but the aspirate-guiding apparatus 200 may be integrated with the suction tube 12.

The elastic bag 30 is a member that is composed of an elastic body such as silicone and that has a bag shape such as an elliptical sphere and functions as the intake apparatus that enables manual ventilation. The elastic bag 30 has the end that has the discharge port 30a through which air in the elastic bag 30 is discharged to the outside and the other end that has the outdoor air introduction port 30b that has the backflow prevention function and through which outdoor air is introduced inward in a direction. The elastic bag 30 that is thus configured is manually compressed and restored, and consequently, the positive pressure ventilation can be performed.

As for the aspirate-suctioning system 2 according to the present embodiment, the intake apparatus that is connected to the intake port 204 is not limited to the elastic bag 30 such as a bag valve mask that corresponds to a manual intake apparatus that assists manual ventilation. For example, an inelastic bag such as a Jackson Rees bag may be used. The intake apparatus can be an invasive positive pressure intake apparatus artificial respirator for, for example, tracheal intubation or a mechanical intake apparatus such as a noninvasive mask artificial respirator such as NPPV (Noninvasive Positive Pressure Ventilation).

According to the present embodiment, as illustrated in, for example, Fig. 12, the patient connection port 203 of the aspirate-guiding apparatus 200 is inserted into the incision hole A2 that is formed in the throat of the patient P1 and is connected to the tracheal cannula 20 that is fixed by using the fixing plate 22 and the cuff 24. At this time, as for the aspirate-guiding apparatus 200, the suction tube 12 of the suction apparatus 10 is connected to the connection tube 201, and the elastic bag 30 is connected to the intake port 204. The patient connection port 203 of the aspirate-guiding apparatus 200 is connected to the end 20a of the tracheal cannula 20, and the positive pressure ventilation is subsequently started by using the elastic bag 30 in order to improve the oxygenation and atelectasis of the lung of the patient.

After the positive pressure ventilation ends, the pressing portion 206a of the operation unit 206 is pressed, the connection source of the patient connection port 203 is consequently switched from the intake port 204 to the connection tube 201 that corresponds to the connection portion of the casing 202, and the suctioning operation is started by using the suction apparatus 10. The suction force (negative pressure) from the suction apparatus 10 is transmitted to the tracheal cannula 20 via the aspirate-guiding apparatus 200, and consequently, vacuuming (guidance) for moving the aspirate such as sputum in the lower respiratory tract to the upper respiratory tract is performed.

That is, as for the aspirate-suctioning system 2 and the method of guiding the aspirate according to the present embodiment, the patient connection port 203 of the aspirate-guiding apparatus 200 is connected to the end 20a of the tracheal cannula 20. The elastic bag 30 and the suction tube 12 of the suction apparatus 10 are connected to the aspirate-guiding apparatus 200. The patient connection port 203 is connected to the end 20a of the tracheal cannula 20, and consequently, the positive pressure ventilation is performed by using the positive pressure from the elastic bag 30 for the lung of the patient P1. After the positive pressure ventilation ends, the pressing portion 206a of the operation unit 206 is pressed, and consequently, the connection source of the patient connection port 203 is switched from the intake port 204 that is connected to the elastic bag 30 to the connection tube 201 that is connected to the suction tube 12.

The aspirate-guiding apparatus 200 is connected also to the suction tube 12. As for the aspirate-guiding apparatus 200, the patient connection port 203 is connected to the end 20a of the tracheal cannula 20. For this reason, the operation unit 206 performs the operation to switch the connection source of the patient connection port 203, and consequently, the tracheal cannula 20 causes the aspirate such as sputum to move to the upper respiratory tract by using the suction force (negative pressure) from the suction apparatus 10. Subsequently, the aspirate such as sputum in the trachea A1 that is guided to the upper respiratory tract is suctioned by the suction catheter.

After suctioning the aspirate such as sputum in the trachea A1 ends, the operation to press the pressing portion 206a of the operation unit 206 is stopped. As a result, the connection source of the patient connection port 203 is switched from the connection tube 201 that is connected to the suction tube 12 to the intake port 204 that is connected to the elastic bag 30, the positive pressure ventilation from the suction apparatus 10 is performed again, and the state of the lung returns to a normal state in which air is present. The aspirate-suctioning system 2 according to the present embodiment can thus easily perform the operation to switch to the positive pressure ventilation that is performed before and after the suction treatment in which the entire lung is contracted by vacuuming under negative pressure by using the operation unit 206 of the aspirate-guiding apparatus 200.

As for the aspirate-suctioning system 2 and the method of guiding the aspirate according to the present embodiment as described above, the aspirate-guiding apparatus 200 performs vacuuming, and the aspirate that is moved to the upper respiratory tract in the trachea A1 by using the suction instrument such as a suction catheter is subsequently suctioned. That is, according to the present embodiment, the aspirate-guiding apparatus 200 is used as an attachment that guides, to a portion of the trachea A1 near the upper respiratory tract, the aspirate such as sputum in the lower respiratory tract that extends from the trachea A1 to the terminal bronchioles in order to make the aspirate likely to be suctioned by using the suction instrument such as a suction catheter. The aspirate-guiding apparatus 200 is also used as an operation member for easily switching to the positive pressure ventilation that is performed before and after vacuuming by performing the operation to press the operation unit 206 with the patient connection port 203 connected to the end 20a of the tracheal cannula 20.

A sputum suctioning method that can be performed by using the aspirate-suctioning system 2 will now be described as an example of the method of guiding the aspirate according to the present embodiment. When the aspirate such as sputum is suctioned from the lower respiratory tract of the patient P1, the lung of the patient P1, for example, is auscultated by using a stethoscope, and the lung is subsequently inflated by the positive pressure ventilation for 5 seconds under a positive pressure of 40 cmH₂O by using the intake apparatus such as an elastic bag. At this time, if a peripheral portion away from a position at which the aspirate is stored is atelectasis, it is difficult to lift the aspirate up to the upper respiratory tract even by vacuuming in some cases because there is no air at the lung periphery. For this reason, the positive pressure ventilation is performed before the suction treatment.

In many cases where the sputum suctioning is needed, the state of the respiration of the patient P1 is bad, blood oxygen saturation is low, the patient P1 cannot breathe, and hypoxia is likely to occur during the sputum suctioning. In view of this, according to the present embodiment, the positive pressure ventilation is performed with oxygen mixed, the condition of the patient is stabilized, and the suction treatment is subsequently started in the case where the positive pressure ventilation described above is performed before the suction treatment. The positive pressure ventilation may be performed with high concentration of oxygen mixed in order to stabilize the condition of the patient P1 in a short time. If the positive pressure ventilation does not need to be performed in a short time, the positive pressure ventilation may be performed by using normal air containing oxygen. A pressure ventilator (an intake apparatus) such as an elastic bag can be included in the aspirate-suctioning system 2 according to the present embodiment.

Subsequently, vacuuming is performed, for example, under a negative pressure of 60 cmH₂O, that is, a pressure of -60 cmH₂O for 5 seconds, the entire lung is contracted, the aspirate such as sputum is guided to the upper respiratory tract, and the aspirate such as sputum that is guided to the upper respiratory tract is subsequently suctioned by the suction instrument such as a suction catheter. At this time, the lung is contracted by vacuuming, and the atelectasis consequently occurs. Accordingly, the positive pressure ventilation is performed again under a positive pressure of 40 cmH₂O for 5 seconds, and the state of the lung consequently returns to a normal state in which air is present. The aspirate-suctioning system 2 according to the present embodiment thus performs the positive pressure ventilation before and after the suction treatment in which the entire lung is contracted by vacuuming under negative pressure.

In the method of guiding the aspirate by using the aspirate-suctioning system 2 according to the present embodiment, the patient P1 is auscultated, a cycle of the positive pressure ventilation, vacuuming under negative pressure, suctioning the aspirate that is guided to the upper respiratory tract, and subsequently performing the positive pressure ventilation again is repeated until the symptoms of the patient P1 are improved. The aspirate-suctioning system 2 according to the present embodiment thus connects the aspirate-guiding apparatus 200 to the end of the suction tube 12 of the suction apparatus 10 and subsequently performs the vacuuming operation for guiding the aspirate in the lower respiratory tract to the upper respiratory tract. The sputum suctioning is easily performed at a low degree of invasion by performing the suctioning operation to suction the aspirate that is guided by the vacuuming operation to the upper respiratory tract by using the suction instrument such as a suction catheter.

According to the present embodiment, the positive pressure ventilation is performed before and after the suctioning operation, the sputum suctioning is consequently performed at a low degree of invasion, and the pain of the patient P1 is reduced. In particular, according to the present embodiment, when the positive pressure ventilation is performed before and after the suctioning operation for vacuuming under negative pressure, the operation to press the operation unit 206 is performed with the patient connection port 203 of the aspirate-guiding apparatus 200 connected to the end 20a of the tracheal cannula 20. The pressing operation is thus performed, and consequently, the operation to switch between vacuuming under negative pressure and the positive pressure ventilation are easily performed. For this reason, foreign substances such as virus and germs are inhibited from entering from the outside during the operation to switch, and the closed sputum suctioning can be more hygienically performed.

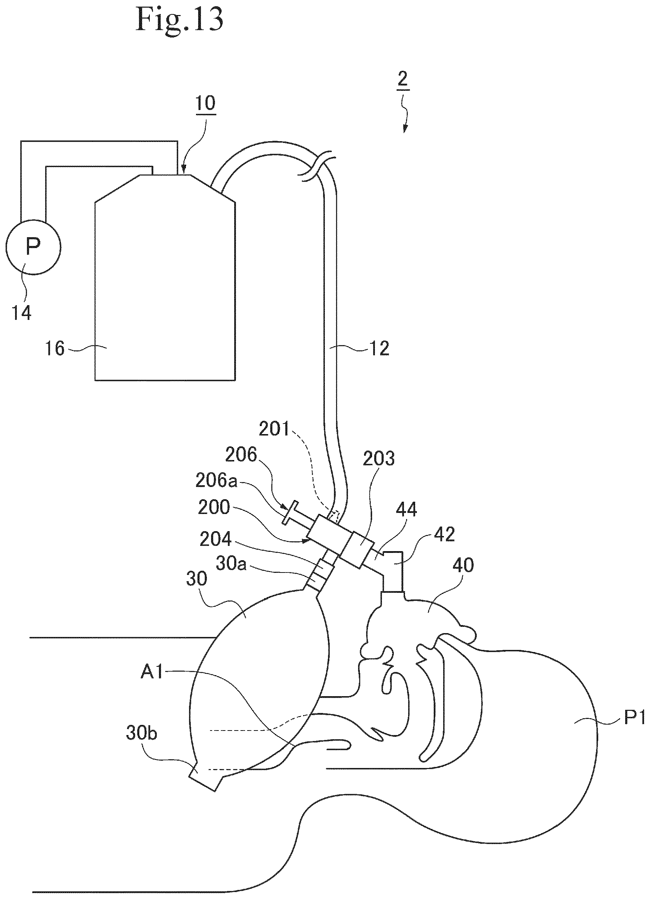

As for the aspirate-suctioning system 2 according to the present embodiment, the aspirate-guiding apparatus 200 that is connected to the suction tube 12 is worn at the throat of the patient P1, and the patient connection port 203 is connected to the tracheal cannula 20 for vacuuming, and the aspirate that is guided to the upper respiratory tract is subsequently suctioned by, for example, the suction catheter, but the aspirate-suctioning system 2 can be used in other aspects. That is, the structure of the aspirate-suctioning system 2 according to the present embodiment may be as illustrated in Fig. 13 even when the wearable ventilation member such as the tracheal cannula 20 is not inserted.

Fig. 13 illustrates an embodiment for the aspirate-guiding apparatus 200, the aspirate-suctioning system 2, and the method of guiding the aspirate by using the aspirate-suctioning system 2. Specifically, according to the embodiment illustrated in Fig. 13, the aspirate-guiding apparatus 200 that connects the suction tube 12 of the suction apparatus 10 and the elastic bag 30 to each other is connected to the branch port 44 that branches from the connection member 42 that is installed at the end portion of the intake mask 40 that covers the oral cavity of the patient P1, and the aspirate is guided. An example of the intake mask 40 can be an oronasal mask.

According to the present embodiment, the patient connection port 203 of the aspirate-guiding apparatus 200 is connected to the branch port 44 of the connection member 42 of the intake mask 40. The negative pressure from the suction apparatus 10 is transmitted due to the connection in this way, and the aspirate such as sputum in the lower respiratory tract that extends from the trachea A1 to the terminal bronchioles is vacuumed toward the upper respiratory tract. For this reason, the aspirate can be guided into the oral cavity of the upper respiratory tract, and accordingly, it is easy that the aspirate that is guided into the oral cavity of the upper respiratory tract can be likely to be suctioned by the suction instrument such as a suction catheter. According to the present embodiment, vacuuming is performed by using the intake mask 40, and consequently, the aspirate in the nasal cavity can be guided to the intake mask 40 for suctioning in addition to the aspirate in the oral cavity.

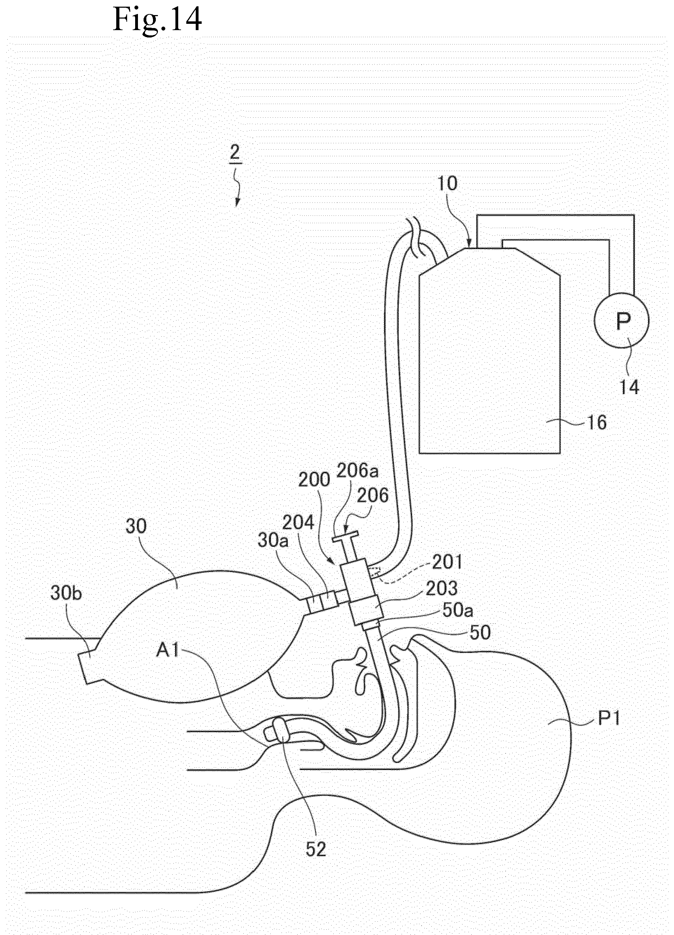

The structure of the aspirate-suctioning system 2 according to the present embodiment may be as illustrated in Fig. 14, which corresponds to an embodiment for the aspirate-guiding apparatus 200, the aspirate-suctioning system 2, and the method of guiding the aspirate by using the aspirate-suctioning system 2. That is, as illustrated in Fig. 14, the aspirate-suctioning system 2 according to the present embodiment is used when the aspirate such as sputum is suctioned and removed from the patient P1 under artificial respirator management in which the tracheal tube 50 is inserted from the mouth of the patient P1 into the trachea A1, for example, in an ICU after surgery.

Specifically, the tracheal tube 50 is inserted toward the trachea A1, and the end of the tracheal tube 50 is fixed by using the cuff 52, and the aspirate-guiding apparatus 200 is connected to the joint 50a that is located at the base end of the tracheal tube 50. The aspirate-guiding apparatus 200 is thus connected, and the negative pressure from the suction apparatus 10 is consequently transmitted such that vacuuming for guiding the aspirate such as sputum to the upper respiratory tract is performed. The aspirate that is guided to the upper respiratory tract is suctioned by the suction instrument such as a suction catheter, and consequently, the aspirate is removed.

The patient connection port 203 of the aspirate-guiding apparatus 200 is thus connected to the joint 50a of the tracheal tube 50, the negative pressure from the suction apparatus 10 is consequently transmitted, and the aspirate such as sputum in the lower respiratory tract is vacuumed. For this reason, the aspirate can be guided to the upper respiratory tract, and accordingly, it is easy that the aspirate that is guided to the upper respiratory tract can be likely to be suctioned by the suction instrument such as a suction catheter.

According to the present embodiment, the operation to press the operation unit 206 is performed with the patient connection port 203 connected to the joint 50a of the tracheal tube 50 when the positive pressure ventilation is performed before and after the suctioning operation, and consequently, the operation to switch between vacuuming and the positive pressure ventilation can be easily performed. For this reason, foreign substances such as virus and germs are inhibited from entering from the outside, and the closed sputum suctioning can be more hygienically performed.

The actions and effects of the aspirate-guiding apparatus 200 and the aspirate-suctioning system 1 according to another embodiment of the present disclosure will now be described.

The aspirate-guiding apparatus 200 according to the present embodiment is connectable to the suction apparatus 10 and the intake apparatus (the elastic bag 30), and the operation to switch the connection source of the patient connection port 203 can be easily performed by the operation unit 206. The patient connection port 203 is connected to the wearable ventilation member, the pressing portion 206a of the operation unit 206 is subsequently pressed, and consequently, the operation to switch the connection source of the patient connection port 203 is performed. Accordingly, the positive pressure ventilation and vacuuming can be easily switched. Consequently, according to the present embodiment, foreign substances such as virus and germs are inhibited from entering from the outside, and the aspirate can be more hygienically performed in a closed manner at a low degree of invasion.

That is, according to the present embodiment, the patient connection port 203 of the aspirate-guiding apparatus 200 that is connected to the suction apparatus 10 and the intake apparatus is connected to the wearable ventilation member such as the tracheal tube 50, the tracheal cannula 20, or the intake mask 40 that is worn on the patient P1. The operation to press the pressing portion 206a of the operation unit 206 is performed, and consequently, the connection source of the patient connection port 203 is switched from the intake port 204 to the suction tube connection hole 201b of the connection tube 201.

The connection source of the patient connection port 203 is thus switched to the suction apparatus 10 that is connected to the connection tube 201 and is in communication with the wearable ventilation member, and consequently, the aspirate is guided to the upper respiratory tract. For this reason, an easy manual operation enables the aspirate to be guided to the upper respiratory tract, and accordingly, the aspirate can be easily suctioned (discharged to the outside from the body) from the upper respiratory tract at a low degree of invasion.

According to the present embodiment, the connection tube 201 of the aspirate-guiding apparatus 200 is inserted into and connected to the end of the suction tube 12, and adhesion between the suction tube 12 and the connection tube 201 is consequently ensured. The patient connection port 203 of the aspirate-guiding apparatus 200 is connected to the wearable ventilation member such as the tracheal cannula 20 or the tracheal tube 50. Accordingly, according to the present embodiment, a practitioner who is not skilled even exerts the same effects of vacuuming as those by a skilled practitioner, and anybody can easily perform vacuuming in the same manner.

According to the present embodiment, the connection tube 201 of the aspirate-guiding apparatus 200 is mounted at the end of the suction tube 12 and is subsequently used as an attachment that is connected to the wearable ventilation member such as the tracheal cannula 20 or the tracheal tube 50 that is worn on the patient P1. The use of the aspirate-guiding apparatus 200 in this way enables the adhesion between the aspirate-guiding apparatus 200 and the suction tube 12 to be ensured and enables the aspirate-guiding apparatus 200 and the wearable ventilation member to be connected to each other. For this reason, the suction force (negative pressure) from the suction apparatus 10 does not leak from a portion at which the suction tube 12 and the connection tube 201 of the aspirate-guiding apparatus 200 are in close contact with each other but is transmitted to the wearable ventilation member such as the tracheal cannula 20 or the tracheal tube 50.

That is, according to the present embodiment, the connection tube 201 of the aspirate-guiding apparatus 200 is connected to the end of the suction tube 12. The pressing portion 206a of the operation unit 206 is pressed with the patient connection port 203 of the aspirate-guiding apparatus 200 connected to the wearable ventilation member such as the tracheal cannula 20 or the tracheal tube 50 that is worn on the patient P1. The pressing portion 206a is thus pressed, the connection source of the patient connection port 203 is switched to the connection tube 201 that is connected to the suction apparatus, and the aspirate such as sputum at the periphery in the lower respiratory tract of the patient P1 is consequently moved to the upper respiratory tract by vacuuming via the patient connection port 203. For this reason, the aspirate that is moved to the upper respiratory tract can be easily suctioned and removed by using the suction instrument such as a suction catheter.

In particular, according to the present embodiment, the shaft 206b that is included in the operation unit of the aspirate-guiding apparatus 200 is a resin tubular member that has the through-hole 206b1 in the axial direction. For this reason, vacuuming is performed by using the aspirate-guiding apparatus 200, and consequently, the aspirate such as sputum at the periphery in the lower respiratory tract of the patient P1 is moved to the upper respiratory tract. The aspirate is moved to the upper respiratory tract, the lid member 206a2 of the pressing portion 206a is subsequently opened, the suction instrument such as a suction catheter is inserted from the opening portion 206a1 so as to extend through the through-hole 206b1, and the aspirate is easily suctioned and removed. After suctioning and removing the aspirate end, the lid member 206a2 is closed with respect to the opening portion 206a1, and consequently, foreign substances such as virus and germs are prevented from entering from the outside, and the operation for suctioning and removing can be more hygienically performed.

According to the present embodiment, the positive pressure ventilation is performed by the intake apparatus such as the elastic bag 30 before the vacuuming operation under negative pressure and after the operation to suction the aspirate that is guided to the upper respiratory tract, and consequently, the aspirate such as sputum can be easily suctioned and removed at a low degree of invasion as described above. In particular, according to the present embodiment, the operation to switch the connection source of the patient connection port 203 of the aspirate-guiding apparatus 200 that is connected to the suction apparatus 10 and the intake apparatus can be easily performed by pressing the pressing portion 206a of the operation unit 206. For this reason, foreign substances such as virus and germs are inhibited from entering from the outside, and the closed sputum suctioning can be more hygienically performed.

According to the present embodiment, the aspirate-guiding apparatus 200 is mounted at the end of the suction tube 12 and is used as an attachment that is connected to the wearable ventilation member that is worn on the patient P1. The aspirate-guiding apparatus 200 is thus used, and consequently, the aspirate can be easily guided to the upper respiratory tract. Accordingly, the aspirate that is guided to the upper respiratory tract is likely to be suctioned, and the efficiency of suctioning is improved. In particular, a method of blindly inserting the suction catheter deeply into the respiratory tract of the patient P1 and provoking a cough during the sputum suctioning gives the patient P1 pain and carries the risk of serious complication such as a cerebrovascular accident due to damage to the respiratory tract or increased blood pressure. According to the present embodiment, however, the pain and the risk of complication can be reduced.

According to the present embodiment, the sputum suctioning can be efficiently performed without using an expensive apparatus that performs the mechanical insufflation exsufflation (MI-E) as in an embodiment described above. For this reason, the pain of the patient P1, for example, is reduced at a decreased cost, and the aspirate such as sputum is suctioned at a low degree of invasion such that the QOL (Quality Of Life) of the patient P1 is improved. According to the present embodiment, the aspirate-guiding apparatus 200 has a simple structure. For this reason, the aspirate-guiding apparatus 200 can be mass-produced at a lower cost than that of a sputum suctioning method (for example, a tracheal endoscope or a percussion ventilator) in which the mechanical insufflation exsufflation (for example, MI-E) or apparatus is used, and the time required for preparation and a cleanup can be reduced.

According to the present embodiment, the aspirate can be guided in a short time with certainty unlike a sputum suctioning method such as postural drainage or squeezing (manual respiration assistance). In particular, in the case of a long-term bedridden patient, the postural drainage and squeezing carries a fracture risk due to a position change or locally manual compression. According to the present embodiment, however, the effects of suctioning the aspirate are exerted by vacuuming regardless of the posture of the patient, and accordingly, the fracture risk due to the position change or the locally manual compression can be reduced.

The embodiments of the present disclosure are described in detail above. A person skilled in the art can easily understand that many modifications can be made without substantially departing from new matters and effects according to the present disclosure. Accordingly, all of the modifications are included in the range of the present disclosure.

For example, a word that is written at least once in the specification or the drawings together with a different word that is more broadly defined or that has the same meaning can be replaced with the different word at any position in the specification or the drawings. The structures and motions of the aspirate-guiding tubes and the aspirate-suctioning systems are not limited to those described according to the embodiments of the present disclosure and can be modified in various ways.

### Reference Signs List

1 aspirate-suctioning system, 10 suction apparatus, 12 suction tube, 14 suction pump, 16 container portion, 20 tracheal cannula, 20a end, 22 fixing plate, 24 cuff, 30 elastic bag (intake apparatus), 30a discharge port, 30b outdoor air introduction port, 40 suction mask, 42 connection member, 44 branch port, 50 tracheal tube, 52 cuff, 100, 200 aspirate-guiding apparatus, 101 aspirate outlet, 101a engagement projection portion, 101b lid portion, 101b1 connecting portion, 102, 202 casing, 103, 203 patient connection port, 104, 204 intake port, 105 connection source switch, 105a switching valve, 105a1 valve member, 105a2 central portion, 105b suction ventilation path, 105c intake ventilation path, 106, 206 operation unit, 106a engagement hole, 106b key wedge, 107 suction catheter, 107a end portion, 107b base end portion, 108 cover member, 108a end portion, 108b base end portion, 108c closed passage, 109 flat spiral spring (elastic member), 110 aspirate-guiding tube, 111 guiding tube body, 112, 201 connection tube (suction connection portion), 112a opening portion, 113 holder, 113a grip portion, 114 inner lid, 114a opening portion, 115 hinge portion, 116 end cap, 117 connection code, 201b suction tube connection hole (aspirate outlet), 202a closing portion, 202a1 opening portion, 202b inner projection portion, 205 sliding portion (switching valve), 205a air vent, 205b side surface, 205c engagement hole, 206a pressing portion, 206a1 opening portion, 206a2 lid member, 206a3 connection code, 206b shaft, 206b1 through-hole, 206c coil spring (elastic member), A1 trachea, A2 incision hole, P1 patient (living body)

## Claims

1. An aspirate-guiding apparatus for guiding an aspirate from a living body to an upper respiratory tract, comprising:
an aspirate outlet through which the aspirate is dischargeable;
a patient connection port that is connectable to a patient;
an intake port that is connectable to an intake apparatus; and
a switching valve that switches a connection source of the patient connection port to the aspirate outlet or the intake port.

2. The aspirate-guiding apparatus according to Claim 1, further comprising:
a casing that includes the aspirate outlet, the patient connection port, and the intake port,
wherein the switching valve is slidably provided in the casing and is capable of switching the connection source of the patient connection port to the aspirate outlet or the intake port.

3. The aspirate-guiding apparatus according to Claim 1 or Claim 2, further comprising:
an elastic member that moves the switching valve such that the connection source of the patient connection port is switched from the aspirate outlet to the intake port.

4. The aspirate-guiding apparatus according to Claim 2 or Claim 3,
wherein in the casing, the aspirate outlet and the patient connection port face each other, and the intake port is provided between the aspirate outlet and the patient connection port,
wherein the switching valve has
a suction ventilation path that is capable of connecting the aspirate outlet and the patient connection port to each other, and
an intake ventilation path that is in communication with the suction ventilation path and that is capable of connecting the intake port and the patient connection port to each other.

5. The aspirate-guiding apparatus according to Claim 4,
wherein the intake ventilation path faces the casing and is closed when the suction ventilation path connects the aspirate outlet and the patient connection port to each other.

6. The aspirate-guiding apparatus according to any one of Claims 3 to 5,
wherein the switching valve is rotatably provided in the casing, and
wherein the elastic member is a flat spiral spring that urges the switching valve rotatably.

7. The aspirate-guiding apparatus according to any one of Claims 3 to 6, further comprising:
an aspirate-guiding tube that includes a suction connection portion that is connected to a suction tube of a suction apparatus,
a suction catheter that is capable of connecting the suction connection portion of the aspirate-guiding tube and the aspirate outlet, and
a cover member that has a closed passage that connects the aspirate-guiding tube and the aspirate outlet to each other and that contains the suction catheter.

8. The aspirate-guiding apparatus according to Claim 2 or Claim 3,
wherein the suction connection portion is provided at a first position on a side surface of the casing,
wherein the patient connection port is provided at a lower end of the casing,
wherein the intake port is provided at a second position on the side surface of the casing, and
wherein the switching valve has an opening at a lower end, has an air vent at an upper end surface, is slidable in the casing, and has a side surface that is capable of closing the intake port or the suction tube connection hole that is located at a base end of the suction connection portion.

9. The aspirate-guiding apparatus according to Claim 8, further comprising:
an operation unit that moves the switching valve along the casing and consequently switches the connection source of the patient connection port,
wherein the operation unit moves the switching valve along the casing between the first position and the second position and consequently switches between a state in which the side surface of the switching valve closes the suction tube connection hole, and the patient connection port and the intake port are in communication with each other such that air is allowed to pass therebetween and a state in which the side surface of the switching valve closes the intake port, and the patient connection port and the suction tube connection hole are in communication with each other such that air is allowed to pass therebetween via the air vent of the switching valve.

10. The aspirate-guiding apparatus according to Claim 8 or Claim 9,
wherein the operation unit includes
a pressing portion that is capable of being pressed from an outside, and
a shaft that connects the pressing portion and the switching valve to each other, and
wherein the operation unit moves the switching valve in and along the casing in a manner in which pressing force to the pressing portion is transmitted to the switching valve via the shaft.

11. The aspirate-guiding apparatus according to Claim 10,
wherein the elastic member is a coil spring that is provided between a closing portion that closes an upper end of the casing and the pressing portion and that applies restoring force in a direction opposite a direction in which the pressing portion is pressed.

12. The aspirate-guiding apparatus according to Claim 10 or claim 11,
wherein the shaft has a through-hole in an axial direction, and
wherein the pressing portion includes an opening portion that is in communication with the through-hole.

13. The aspirate-guiding apparatus according to Claim 12,
wherein the pressing portion includes a lid member that is capable of opening and closing with respect to the opening portion.

14. An aspirate-suctioning system for suctioning an aspirate from a living body, comprising:
a suction apparatus that includes a suction tube;
the aspirate-guiding apparatus according to any one of Claims 1 to 13 that is connected to an end of the suction tube; and
an intake apparatus that is connected to the intake port of the aspirate-guiding apparatus.
